# EUROPEAN PATENT APPLICATION

(11) **EP 3 305 324 A1**
(43) Date of publication of application: **11.04.2018**
(21) Application number: 17196705.2
(22) Date of filing: 31.10.2008
(51) Int. Cl.: A61K 39/395, A61P 35/00, C07K 16/28

(54) **MOLECULES AND METHODS FOR MODULATING LOW-DENSITY-LIPOPROTEIN RECEPTOR-RELATED PROTEIN 6 (LRP6)**

(30) Priority: 02.11.2007 US 984827 P
(62) Divisional of application: 12188230.2
(71) Applicant: Novartis AG, 4056 Basel (CH)
(72) Inventor: CONG, Feng, Quincy, MA 02171 (US); RANGWALA, Shamina M., Brookline, MA 02445 (US); ETTENBERG, Seth, Melrose, MA 02176 (US); GUTH, Sabine, 4057 Basel (CH)
(74) Representative: Carpmaels & Ransford LLP

(57) **Abstract**

The invention discloses LRP6 agonizing or antagonizing binding molecules (e.g., antibodies or a Fab fragments), and their use to facilitate or inhibit Wnt pathway signaling, respectively. Said LRP6 agonizing or antagonizing binding molecules can be used to e.g., diagnose, ameliorate the symptoms of, protect against, and treat Wnt signaling disorders associated with aberrantly low or aberrantly high levels of Wnt pathway signaling, respectively. Non-limiting examples of disorders which can be treated associated with aberrant upregulation of Wnt signaling is cancer (e.g., colon cancer). Non-limiting examples of disorders which can be diagnosed, protected against, and treated include cancers, bone disorders (e.g., osteoporosis and osteoarthritis), diabetes, neurodegenerative diseases such as Alzheimer's disease, and fibrotic disorders.

SEQUENCE LISTING

<110> Cong, Feng
      Ettenberg, Seth A.
      Rangwala, Shamina M.
      Guth, Sabine

<120> MOLECULES AND METHODS FOR MODULATING
  LOW-DENSITY-LIPOPROTEIN RECEPTOR-RELATED PROTEIN 6 (LRP6)

<130> 4-52291

<160> 5

<170> FastSEQ for Windows Version 4.0

<210> 1
<211> 1613
<212> PRT
<213> Homo sapiens

<400> 1

<210> 2
<211> 307
<212> PRT
<213> Homo sapiens

<400> 2
Ala Pro Leu Leu Leu Tyr Ala Asn Arg Arg Asp Leu Arg Leu Val Asp
 1               5                  10                  15
Ala Thr Asn Gly Lys Glu Asn Ala Thr Ile Val Val Gly Gly Leu Glu
            20                  25                  30
Asp Ala Ala Ala Val Asp Phe Val Phe Ser His Gly Leu Ile Tyr Trp
        35                  40                  45
Ser Asp Val Ser Glu Glu Ala Ile Lys Arg Thr Glu Phe Asn Lys Thr
    50                  55                  60
Glu Ser Val Gln Asn Val Val Val Ser Gly Leu Leu Ser Pro Asp Gly
65                  70                  75                  80
Leu Ala Cys Asp Trp Leu Gly Glu Lys Leu Tyr Trp Thr Asp Ser Glu
                85                  90                  95
Thr Asn Arg Ile Glu Val Ser Asn Leu Asp Gly Ser Leu Arg Lys Val
            100                 105                 110
Leu Phe Trp Gln Glu Leu Asp Gln Pro Arg Ala Ile Ala Leu Asp Pro
        115                 120                 125
Ser Ser Gly Phe Met Tyr Trp Thr Asp Trp Gly Glu Val Pro Lys Ile
    130                 135                 140

Glu Arg Ala Gly Met Asp Gly Ser Ser Arg Phe Ile Ile Ile Asn Ser
145                 150                 155                 160
Glu Ile Tyr Trp Pro Asn Gly Leu Thr Leu Asp Tyr Glu Glu Gln Lys
                165                 170                 175
Leu Tyr Trp Ala Asp Ala Lys Leu Asn Phe Ile His Lys Ser Asn Leu
            180                 185                 190
Asp Gly Thr Asn Arg Gln Ala Val Val Lys Gly Ser Leu Pro His Pro
        195                 200                 205
Phe Ala Leu Thr Leu Phe Glu Asp Ile Leu Tyr Trp Thr Asp Trp Ser
    210                 215                 220
Thr His Ser Ile Leu Ala Cys Asn Lys Tyr Thr Gly Glu Gly Leu Arg
225                 230                 235                 240
Glu Ile His Ser Asp Ile Phe Ser Pro Met Asp Ile His Ala Phe Ser
                245                 250                 255
Gln Gln Arg Gln Pro Asn Ala Thr Asn Pro Cys Gly Ile Asp Asn Gly
            260                 265                 270
Gly Cys Ser His Leu Cys Leu Met Ser Pro Val Lys Pro Phe Tyr Gln
        275                 280                 285
Cys Ala Cys Pro Thr Gly Val Lys Leu Leu Glu Asn Gly Lys Thr Cys
    290                 295                 300
Lys Asp Gly
305

<210> 3
<211> 44
<212> PRT
<213> Homo sapiens

<400> 3
Asn Ala Thr Asn Pro Cys Gly Ile Asp Asn Gly Gly Cys Ser His Leu
1               5                   10                  15
Cys Leu Met Ser Pro Val Lys Pro Phe Tyr Gln Cys Ala Cys Pro Thr
            20                  25                  30
Gly Val Lys Leu Leu Glu Asn Gly Lys Thr Cys Lys
        35                  40

<210> 4
<211> 302
<212> PRT
<213> Homo sapiens

<400> 4
Glu Ala Phe Leu Leu Phe Ser Arg Arg Ala Asp Ile Arg Arg Ile Ser
1               5                   10                  15
Leu Glu Thr Asn Asn Asn Asn Val Ala Ile Pro Leu Thr Gly Val Lys
            20                  25                  30
Glu Ala Ser Ala Leu Asp Phe Asp Val Thr Asp Asn Arg Ile Tyr Trp
        35                  40                  45
Thr Asp Ile Ser Leu Lys Thr Ile Ser Arg Ala Phe Met Asn Gly Ser
    50                  55                  60
Ala Leu Glu His Val Val Glu Phe Gly Leu Asp Tyr Pro Glu Gly Met
65                  70                  75                  80
Ala Val Asp Trp Leu Gly Lys Asn Leu Tyr Trp Ala Asp Thr Gly Thr
                85                  90                  95
Asn Arg Ile Glu Val Ser Lys Leu Asp Gly Gln His Arg Gln Val Leu
            100                 105                 110
Val Trp Lys Asp Leu Asp Ser Pro Arg Ala Leu Ala Leu Asp Pro Ala
        115                 120                 125
Glu Gly Phe Met Tyr Trp Thr Glu Trp Gly Gly Lys Pro Lys Ile Asp
    130                 135                 140
Arg Ala Ala Met Asp Gly Ser Glu Arg Thr Thr Leu Val Pro Asn Val

145                 150                 155                 160
Gly Arg Ala Asn Gly Leu Thr Ile Asp Tyr Ala Lys Arg Arg Leu Tyr
                165                 170                 175
Trp Thr Asp Leu Asp Thr Asn Leu Ile Glu Ser Ser Asn Met Leu Gly
            180                 185                 190
Leu Asn Arg Glu Val Ile Ala Asp Asp Leu Pro His Pro Phe Gly Leu
        195                 200                 205
Thr Gln Tyr Gln Asp Tyr Ile Tyr Trp Thr Asp Trp Ser Arg Arg Ser
    210                 215                 220
Ile Glu Arg Ala Asn Lys Thr Ser Gly Gln Asn Arg Thr Ile Ile Gln
225                 230                 235                 240
Gly His Leu Asp Tyr Val Met Asp Ile Leu Val Phe His Ser Ser Arg
                245                 250                 255
Gln Ser Gly Trp Asn Glu Cys Ala Ser Ser Asn Gly His Cys Ser His
            260                 265                 270
Leu Cys Leu Ala Val Pro Val Gly Gly Phe Val Cys Gly Cys Pro Ala
        275                 280                 285
His Tyr Ser Leu Asn Ala Asp Asn Arg Thr Cys Ser Ala Pro
    290                 295                 300

<210> 5
<211> 41
<212> PRT
<213> Homo sapiens

<400> 5
Gly Trp Asn Glu Cys Ala Ser Ser Asn Gly His Cys Ser His Leu Cys
 1               5                  10                  15
Leu Ala Val Pro Val Gly Gly Phe Val Cys Gly Cys Pro Ala His Tyr
            20                  25                  30
Ser Leu Asn Ala Asp Asn Arg Thr Cys
        35                  40

## Description

### BACKGROUND OF THE INVENTION

The Wnt gene family encodes a large class of secreted proteins related to the Int1/Wnt1 proto-oncogene and Drosophila wingless ("Wg"), a Drosophila Wnt1 homologue (Cadigan et al. (1997) Genes & Development 11:3286-3305). Wnts are expressed in a variety of tissues and organs and are required for many developmental processes, including segmentation in Drosophila; endoderm development in C. elegans; and establishment of limb polarity, neural crest differentiation, kidney morphogenesis, sex determination, and brain development in mammals (Parr, et al. (1994) Curr. Opinion Genetics & Devel. 4:523-528). The Wnt pathway is a master regulator in animal development, both during embryogenesis and in the mature organism (Eastman, et al. (1999) Curr Opin Cell Biol 11: 233-240; Peifer, et al. (2000) Science 287: 1606-1609).

Wnt signals are transduced by the Frizzled ("Fz") family of seven transmembrane domain receptors (Bhanot et al. (1996) Nature 382:225-230). Frizzled cell-surface receptors (Fzd) play an essential role in both canonical and non-canonical Wnt signaling. In the canonical pathway, upon activation of Fzd and LRP5/6 (low-density-lipoprotein receptor-related protein 5 and 6) by Wnt proteins, a signal is generated that prevents the phosphorylation and degradation of β-catenin by the "β-cat destruction complex," permitting stable β-catenin translocation and accumulation in the nucleus, and therefore Wnt signal transduction. (Perrimon (1994) Cell 76:781-784)(Miller, J.R. (2001) Genome Biology; 3(1):1-15). The non-canonical Wnt signaling pathway is less well defined: there are at least two non-canonical Wnt signaling pathways that have been proposed, including the planar cell polarity (PCP) pathway, the Wnt/Ca++ pathway, and the convergence extension pathway.

Glycogen synthase kinase 3 (GSK3, known as shaggy in Drosophila), the tumor suppressor gene product APC (adenomatous polyposis coli)(Gumbiner (1997) Curr. Biol. 7:R443-436), and the scaffolding protein Axin, are all negative regulators of the Wnt pathway, and together form the "β-cat destruction complex." In the absence of a Wnt ligand, these proteins form a complex and promote phosphorylation and degradation of β-catenin, whereas Wnt signaling inactivates the complex and prevents β-catenin degradation. Stabilized β-catenin translocates to the nucleus as a result, where it binds TCF (T cell factor) transcription factors (also known as lymphoid enhancer-binding factor-1 (LEF1)) and serves as a coactivator of TCF/LEF-induced transcription (Bienz, et al. (2000) Cell 103: 311-320; Polakis, et al. (2000) Genes Dev 14: 1837-1851).

Wnt signaling occurs via canonical and non-canonical mechanisms. In the canonical pathway, upon activation of Fzd and LRP5/6 by Wnt proteins, stabilized β-catenin accumulates in the nucleus and leads to activation of TCF target genes (as described above). (Miller, J.R. (2001) Genome Biology; 3(1):1-15) The non-canonical Wnt signaling pathway is less well defined: at least two non-canonical Wnt signaling pathways have been proposed, including the planar cell polarity (PCP) pathway and the Wnt/Ca++ pathway.

Aberrantly high Wnt pathway activation, through the stabilization of β-catenin, plays a central role in tumorigenesis for many colorectal carcinomas. It is estimated that 80% of colorectal carcinomas (CRCs) harbor inactivating mutations in the tumor repressor APC, which allows for uninterrupted Wnt signaling. Furthermore, there is a growing body of evidence that suggests that Wnt-pathway activation may be involved in melanoma, breast, liver, lung, and gastric cancers. There is a long-recognized connection between Wnts, normal development, and cancer, a connection further established with the identification the c-Myc proto-oncogene as a target of Wnt signaling (He et al. (1998) Science 281:1509-3512).

Furthermore, other disorders associated with pathologically high or low levels of Wnt signaling include, but are not limited to, osteoporosis, osteoarthritis, polycystic kidney disease, diabetes, schizophrenia, vascular disease, cardiac disease, non-oncogenic proliferative diseases, and neurodegenerative diseases such as Alzheimer's disease. Aberrant upregulation of Wnt signaling is associated with cancer, osteoarthritis, and polycystic kidney disease, whereas aberrant downregulation of Wnt signaling has been linked to osteoporosis, obesity, diabetes, fibrotic disorders, and neuronal degenerative diseases.

The current paradigm for developing therapies for Wnt signaling-related disorders, such as colorectal cancer, relies on targeting β-cat or Wnt pathway components downstream of β-cat. Recent studies, however, suggest that autocrine Wnt signaling mediated by Wnt receptor Frizzled and LRP5/6 may play key roles in regulating tumor growth and survival. A need exists for agents and methods that inhibit or potentiate Wnt signal transduction activity by modulating activation of the Wnt pathway along other critical junctures, thereby treating, diagnosing, preventing, and/or ameliorating Wnt signaling-related disorders.

### SUMMARY OF THE INVENTION

The present invention relates to epitopes of LRP6, LRP6 binding molecules, and methods of using the molecules. LRP6 binding molecules interact with LRP6 and are thereby capable of modulating LRP6 functions. LRP6 agonizing or antagonizing binding molecules can be used to facilitate or inhibit Wnt pathway signaling, respectively; therefore, LRP6 agonizing or antagonizing binding molecules can be used to e.g., diagnose, ameliorate the symptoms of, protect against, and treat Wnt signaling disorders associated with aberrantly low or aberrantly high levels of Wnt pathway signaling, respectively. A non-limiting example of a disorder associated with aberrant upregulation of Wnt signaling is cancer (e.g., breast cancer, lung cancer, and colon cancer). Non-limiting examples of disorders associated with aberrant downregulation of Wnt signaling are bone-related disorders characterized by low bone mineral density (BMD)(e.g., osteoporosis).

In various aspects, the invention provides LRP6 binding molecules that modulate (e.g., inhibit or promote) one or more biological functions of LRP6. For example, an LRP6 binding molecule can modulate phosphorylation and subsequent degradation of β-catenin. By way of further example, an LRP6 binding molecule can interfere with LRP6's ability to bind to integral members of the Wnt pathway, e.g., DKK1 (dickkopf 1), DKK2, DKK4, SOST1, SOSD1 1 (USAG1), sFRP (soluble Fzd-related protein) 1-4, Wise (the mouse version of USAG1), or Wnt ligands themselves.

LRP6 binding molecules include, for example, antibodies that bind to LRP6 (e.g., within a particular domain or epitope of LRP6, such to the first or third propellers of the extracellular domain of LRP6, or to specific domains within any propeller (e.g., an EGF repeat, a YWTD-like motif, or others)), and polypeptides that include antigen binding portions of such antibodies. LRP6 binding molecules also include molecules in which the binding portion is not derived from an antibody, e.g., LRP6 binding molecules derived from polypeptides that have an immunoglobulin-like fold, and in which the antigen binding portion is engineered to bind LRP6 through randomization, selection, and affinity maturation.

Accordingly, in one aspect, the invention features an LRP6 binding molecule including an antigen binding portion of an antibody that binds (e.g., specifically binds) to LRP6, wherein the antigen binding portion binds to an epitope within propeller 1 of human LRP6 (SEQ ID NO:2; residues 20-326 of SEQ ID NO:1) within or overlapping one of the following: (a) amino acids 286-324 of SEQ ID NO:1 (i.e., an epitope within or overlapping the following sequence:

### NATNPCGIDN GGCSHLCLMS PVKPFYQCAC PTGVKLLENG KTCK (SEQ ID NO:3)); (b)

amino acids 66-69 of SEQ ID NO:1 (i.e., an epitope within or overlapping the following sequence: YWSD); (c) amino acids 110-113 of SEQ ID NO:1 (i.e., an epitope within or overlapping the following sequence: YWTD); (d) amino acids 153-156 of SEQ ID NO:1 (i.e., an epitope within or overlapping the following sequence: YWTD); (e) amino acids 197-200 of SEQ ID NO:1 (i.e., an epitope within or overlapping the following sequence: YWAD); and (f) amino acids 238-241 of SEQ ID NO:1 (i.e., an epitope within or overlapping the following sequence: YWTD).

In another aspect, the invention features an LRP6 binding molecule including an antigen binding portion of an antibody that binds (e.g., specifically binds) to LRP6, wherein the antigen binding portion binds to an epitope within propeller 1 of human LRP6 (SEQ ID NO:2; residues 20-326 of SEQ ID NO:1) within or overlapping one of the following: (a) amino acids 20-65 of SEQ ID NO:1; (b) amino acids 70-109 of SEQ ID NO:1; (c) amino acids 114-152 of SEQ ID NO:1; (d) amino acids 157-196 of SEQ ID NO:1; and (e) amino acids 201-237 of SEQ ID NO:1; and (f) amino acids 242-326 of SEQ ID NO:1.

Accordingly, in one aspect, the invention features an LRP6 binding molecule including an antigen binding portion of an antibody that binds (e.g., specifically binds) to LRP6, wherein the antigen binding portion binds to an epitope within propeller 3 of human LRP6 (SEQ ID NO:4; residues 631-932 of SEQ ID NO:1) within or overlapping one of the following: (a) amino acids 889-929 of SEQ ID NO:1 (i.e., an epitope within or overlapping the following sequence: GW NECASSNGHC SHLCLAVPVG GFVCGCPAHY SLNADNRTC (SEQ ID NO:5)); (b) amino acids 677-680 of SEQ ID NO:1 (i.e., an epitope within or overlapping the following sequence: YWTD); (c) amino acids 720-723 of SEQ ID NO:1 (i.e., an epitope within or overlapping the following sequence: YWAD); (d) amino acids 763-766 of SEQ ID NO:1 (i.e., an epitope within or overlapping the following sequence: YWTE); (e) amino acids 806-809 of SEQ ID NO:1 (i.e., an epitope within or overlapping the following sequence: YWTD); and (f) amino acids 846-849 of SEQ ID NO:1 (i.e., an epitope within or overlapping the following sequence: YWTD).

In another aspect, the invention features an LRP6 binding molecule including an antigen binding portion of an antibody that binds (e.g., specifically binds) to LRP6, wherein the antigen binding portion binds to an epitope within propeller 3 of human LRP6 (SEQ ID NO:4; residues 631-932 of SEQ ID NO:1) within or overlapping one of the following: (a) amino acids 631-676 of SEQ ID NO:1; (b) amino acids 681-719 of SEQ ID NO:1; (c) amino acids 724-762 of SEQ ID NO:1; (d) amino acids 767-805 of SEQ ID NO:1; (e) amino acids 810-845 of SEQ ID NO:1; and (f) amino acids 850-932 of SEQ ID NO:1.

In various embodiments, the LRP6 binding molecule (e.g., an anti-LRP6 antagonizing antibody) binds in such a way to an epitope within human LRP6 (e.g., to human LRP6 propeller 1, propeller 3, or to constituent domains or motifs thereof) so as to prevent certain Wnt ligands from similarly binding. By way of non-limiting example, LRP6 binding molecules which antagonize Wnt signaling pathway prevent Wnt1 or Wnt3a ligands from binding LRP6. For example, Wnt3- and Wnt3a-specific signaling activity can be most effectively inhibited by a Wnt3a specific LRP6 antagonizing binding molecule. By way of further non-limiting example, Wnt1-, Wnt2-, Wnt6-, Wnt7a-, Wnt7b-, and Wnt10-specific signaling activity can be most effectively inhibited by a Wnt1 specific LRP6 antagonizing binding molecule.

In various embodiments, the LRP6 binding molecule (e.g., an anti-LRP6 antagonizing antibody) is capable of binding to more than one propeller of LRP6 (e.g., simultaneously to propeller 1, propeller 3, or to constituent domains or motifs thereof)). In some instances, said simultaneously-binding LRP6 binding molecule is capable of preventing certain Wnt ligands from similarly binding LRP6. By way of non-limiting example, said simultaneously-binding LRP6 binding molecules are capable of preventing Wnt1 or Wnt3a ligands from binding LRP6. By way of non-limiting example, said simultaneously-binding LRP6 binding molecules are capable of inhibiting Wnt3- and Wnt3a-specific signaling activity. By way of further non-limiting example, said simultaneously-binding LRP6 binding molecules are capable of inhibiting Wnt1-, Wnt2-, Wnt6-, Wnt7a-, Wnt7b-, and Wnt10-specific signaling activity.

In various embodiments, the LRP6 binding molecule (e.g., an anti-LRP6 agonizing or antagonizing antibody) that binds to an epitope within human LRP6 propeller 1, propeller 3, or within constituent domains or motifs thereof is cross reactive with the LRP6 protein (or portion thereof) of a non-human primate (e.g., a cynomolgus monkey, or a rhesus monkey). In various embodiments, the LRP6 binding molecule is cross reactive with LRP6 of a rodent species (e.g., murine LRP6, rat LRP6). In various embodiments, the LRP6 binding molecule is cross reactive with human LRP5.

In various embodiments, the LRP6 binding molecule (e.g., an anti-LRP6 antagonizing antibody) is capable of binding to and modulating (e.g., inhibiting) phosphorylated-LRP6 (phospho-LRP6).

In various embodiments, the antigen binding portion binds to a linear epitope.

In various embodiments, the antigen binding portion binds to a non-linear epitope. In one example, the antigen binding portion binds to a non-linear epitope including, or consisting of, at least one portion of each of the following linear epitopes: (a) amino acids 286-324 of SEQ ID NO:1; (b) amino acids 66-69 of SEQ ID NO:1; (c) amino acids 110-113 of SEQ ID NO:1; (d) amino acids 153-156 of SEQ ID NO:1; (e) amino acids 197-200 of SEQ ID NO:1; and (f) amino acids 238-241 of SEQ ID NO:1. In another example, the antigen binding portion binds to a non-linear epitope including, or consisting of, at least one portion of two or three of the following linear epitopes: (a) amino acids 889-929 of SEQ ID NO:1; (b) amino acids 677-680 of SEQ ID NO:1; (c) amino acids 720-723 of SEQ ID NO:1; (d) amino acids 763-766 of SEQ ID NO:1; (e) amino acids 806-809 of SEQ ID NO:1; and (f) amino acids 846-849 of SEQ ID NO:1.

In various embodiments, the antigen binding portion of the LRP6 binding molecule binds to LRP6 with a dissociation constant (K_{D}) equal to or less than 1 nM, 0.5 nM, 0.25 nM, or 0.1 nM.

In various embodiments, the antigen binding portion of the LRP6 binding molecule binds to LRP6 of a non-human primate (e.g., cynomolgus monkey or chimpanzee) with a K_{D} equal to or less than 1 nM, 0.5 nM, 0.25 nM, or 0.1 nM.

In various embodiments, antigen binding portion binds to mouse LRP6 with a K_{D} equal to or less than 1 nM, 0.5 nM, 0.25 nM, or 0.1 nM.

The antibody can be a chimeric (e.g., humanized) antibody or a human antibody.

In one embodiment, the antigen binding portion is an antigen binding portion of a human antibody.

The antigen binding portion can be an antigen binding portion of a monoclonal antibody or a polyclonal antibody.

The LRP6 binding molecule includes, for example, a Fab fragment, a Fab' fragment, a F(ab')₂, or an Fv fragment of the antibody. The LRP6 binding molecule also includes, for example, complete antibodies, e.g., bivalent IgG antibodies. In some embodiments, a Fab or other monovalent antibody fragment that is capable of antagonizing LRP6 can be converted into a bivalent complete antibody, e.g., a bivalent IgG antibody, which is capable of agonizing LRP6.

In other embodiments, the LRP6 binding molecule is conjugated or coupled with an agent to increase said binding molecule. In some instances, the LRP6 binding molecule is conjugated with human serum albumin (HSA) binding protein(s). In some embodiments, the LRP6 binding molecule is pegylated (e.g., using proprietary technology as described in any of US patent numbers US 5,840,526; US 5,874,541; US 6,005,079; and US 6,765,087 (the "Hamers patents"), and in the patent family which includes PCT publication WO97/49805). In some embodiments, the LRP6 binding molecule is a pegylated Fab fragment. Non-limiting examples of techniques which can be employed to manufacture said conjugates of LRP6 binding molecules can be found in patent families which include one or more of the following: US patent numbers US 5,840,526; US 5,874,541; US 6,005,079; and US 6,765,087 (the "Hamers patents"); PCT publication WO97/49805; European patent EP1517921B1; US patent 6,267,974; and US publication US2003-0175921.

In one embodiment, the LRP6 binding molecule is a human antibody.

In one embodiment, the LRP6 binding molecule includes a single chain Fv.

In one embodiment, the LRP6 binding molecule includes a diabody (e.g., a single chain diabody, or a diabody having two polypeptide chains).

In some embodiments, the antigen binding portion of the antibody is derived from an antibody of one of the following isotypes: IgG1, IgG2, IgG3 or IgG4. In some embodiments, the antigen binding portion of the antibody is derived from an antibody of an IgA or IgE isotype.

The LRP6 binding molecule (e.g., the LRP6 binding molecule that binds to an epitope within the first or third propellers of the extracellular domain of LRP6, or to specific domains within said propellers (e.g., an EGF repeat, or a YWTD-like motif)) can exhibit one or more of a number of biological activities. In various embodiments, the LRP6 binding molecule inhibits LRP6 binding to various Wnt signaling pathway members (e.g., DKK1 (dickkopf 1), DKK2, DKK4, SOST1, SOSD1 (USAG1), sFRP (soluble Fzd-related protein) 1-4, Wise, or Wnt ligands themselves). For example, the LRP6 binding molecule inhibits LRP6 binding to Wnt signaling pathway members by at least 5%, 10%, 15%, 25%, or 50%, relative to a control (e.g., relative to binding in the absence of the LRP6 binding molecule).

In one embodiment, an LRP6 binding molecule competes with Wnt signaling pathway members (e.g., DKK1 (dickkopf 1), DKK2, DKK4, SOST1, SOSD1 (USAG1), sFRP (soluble Fzd-related protein) 1-4, Wise, or Wnt ligands themselves) for binding to LRP6, thereby modulating the biological activity and consequences of Wnt pathway signaling. By way of non-limiting example, an antagonizing LRP6 binding molecule can inhibit, attenuate, or prevent Wnt pathway activation and signaling by competing with Wnt signaling pathway members for binding to LRP6. By way of further example, a Wnt1 specific antagonizing LRP6 binding molecule can prevent Wnt pathway activation and signaling by any of Wnt1, Wnt2, Wnt6, Wnt7a, Wnt7b and Wnt10. By way of further example, an antagonizing LRP6 binding molecule (e.g., which binds within the first propeller of LRP6) can inhibit, attenuate, or prevent Wnt pathway activation and signaling by Wise or Wnt ligands. By way of still further example, an antagonizing LRP6 binding molecule (e.g., which binds within the third propeller of LRP6) can inhibit, attenuate, or prevent Wnt pathway activation and signaling by, e.g., DKK1, and Wnt ligands such as Wnt3 and Wnt3a.

In some embodiments, an LRP6 binding molecule is capable of activating, enhancing, or perpetuating the Wnt signaling pathway, or sensitizing cells to Wnt signaling. By way of non-limiting example, agonizing LRP6 binding molecules are capable of binding to LRP6 (e.g., to the 3rd propeller) and engendering the dissolution of the β-catenin destruction complex, thereby permitting β-catenin stabilization, translocation to the nucleus, and engagement of transcription factors.

In some embodiments, agonizing LRP6 binding molecules are capable of activating, enhancing, or perpetuating the Wnt signaling pathway, or sensitizing cells to Wnt signaling, by oligomerizing LRP6. It is known that the requirement for Wnt ligands can be abrogated by agents which allow formation of oligomerization of LRP6 (e.g., Fzd-LRP6 hetrero-oligomers), including the LRP6 binding molecules of the invention. (Cong et al. (2004) Development 131(20): 5103). In some embodiments, said LRP6 binding molecules are divalent IgG antibodies, as described herein.

In some embodiments, the LRP6 binding molecule modulates downstream biological activities normally modulated in a direct or indirect fashion by LRP6 (e.g., modulation of β-catenin phosphorylation and degradation). For example, an antagonizing LRP6 binding molecule permits β-catenin phosphorylation and degradation by at least a 5%, 10%, 15%, 25%, or 50%, greater margin relative to a control (e.g., relative to activity in the absence of the antagonizing LRP6 binding molecule).

In one embodiment, a Wnt1 specific antagonizing LRP6 binding molecule permits β-catenin phosphorylation and degradation by at least a 5%, 10%, 15%, 25%, or 50%, greater margin relative to a control by preventing Wnt pathway activation and signaling by any of Wnt1, Wnt2, Wnt6, Wnt7a, Wnt7b, and Wnt10. In another embodiment, a Wnt3a specific antagonizing LRP6 binding molecule permits β-catenin phosphorylation and degradation by at least a 5%, 10%, 15%, 25%, or 50%, greater margin relative to a control by preventing Wnt pathway activation and signaling by Wnt3 or Wnt3a. In another embodiment, a Wnt1 specific antagonizing LRP6 binding molecule permits β-catenin phosphorylation and degradation by at least a 5%, 10%, 15%, 25%, or 50%, greater margin relative to a control by preventing Wise from binding to LRP6. In yet another embodiment, a Wnt3a specific antagonizing LRP6 binding molecule permits β-catenin phosphorylation and degradation by at least a 5%, 10%, 15%, 25%, or 50%, greater margin relative to a control by preventing DKK1, Wnt ligands such as Wnt3 and Wnt3a, etc. from binding to LRP6.

Conversely, for example, an agonizing LRP6 binding molecule stabilizes β-catenin protein levels by at least a 5%, 10%, 15%, 25%, or 50%, greater margin relative to a control (e.g., relative to activity in the absence of the agonizing LRP6 binding molecule).

The invention also features non-antibody LRP6 binding molecules. A non-antibody LRP6 binding molecule includes an LRP6 binding domain that has an amino acid sequence derived from an immunoglobulin-like (Ig-like) fold of a non-antibody polypeptide, such as one of the following: tenascin, N-cadherin, E-cadherin, ICAM, fibronectin, titin, GCSF-receptor, cytokine receptor, glycosidase inhibitor, antibiotic chromoprotein, myelin membrane adhesion molecule P0, CD8, CD4, CD2, class I MHC, T-cell antigen receptor, CD1, C2 and I-set domains of VCAM-1, I-set immunoglobulin domain of myosin-binding protein C, I-set immunoglobulin domain of myosin-binding protein H, I-set immunoglobulin domain of telokin, NCAM, twitchin, neuroglian, growth hormone receptor, erythropoietin receptor, prolactin receptor, interferon-gamma receptor, β-galactosidase/glucuronidase, β-glucuronidase, transglutaminase, T-cell antigen receptor, superoxide dismutase, tissue factor domain, cytochrome F, green fluorescent protein, GroEL, or thaumatin. In general, the amino acid sequence of the LRP6 binding domain is altered, relative to the amino acid sequence of the immunoglobulin-like fold, such that the LRP6 binding domain specifically binds to the LRP6 (i.e., wherein the immunoglobulin-like fold does not specifically bind to the LRP6).

In various embodiments, the amino acid sequence of the LRP6 binding domain is at least 60% identical (e.g., at least 65%, 75%, 80%, 85%, or 90% identical) to an amino acid sequence of an immunoglobulin-like fold of a fibronectin, a cytokine receptor, or a cadherin.

In various embodiments, the amino acid sequence of the LRP6 binding domain is at least 60%, 65%, 75%, 80%, 85%, or 90% identical to an amino acid sequence of an immunoglobulin-like fold of one of the following: tenascin, N-cadherin, E-cadherin, ICAM, titin, GCSF-receptor, cytokine receptor, glycosidase inhibitor, antibiotic chromoprotein, myelin membrane adhesion molecule P0, CD8, CD4, CD2, class I MHC, T-cell antigen receptor, CD1, C2 and I-set domains of VCAM-1, I-set immunoglobulin domain of myosin-binding protein C, I-set immunoglobulin domain of myosin-binding protein H, I-set immunoglobulin domain of telokin, NCAM, twitchin, neuroglian, growth hormone receptor, erythropoietin receptor, prolactin receptor, interferon-gamma receptor, β-galactosidase/glucuronidase, β-glucuronidase, transglutaminase, T-cell antigen receptor, superoxide dismutase, tissue factor domain, cytochrome F, green fluorescent protein, GroEL, or thaumatin.

In various embodiments, the LRP6 binding domain binds to LRP6 with a K_{D} equal to or less than 1 nM (e.g., 0.5 nM, 01 nM).

In some embodiments, the Ig-like fold is an Ig-like fold of a fibronectin, e.g., an Ig-like fold of fibronectin type III (e.g., an Ig-like fold of module 10 of fibronectin III).

The invention also provides peptides corresponding to antigenic epitopes of LRP6. In one aspect, the invention features a peptide consisting of an amino acid sequence at least 90% identical to one of following amino acid sequences: the first propeller of LRP6 (SEQ ID NO:2); NATNPCGIDN GGCSHLCLMS PVKPFYQCAC PTGVKLLENG KTCK (SEQ ID NO:3); the third propeller of LRP6 (SEQ ID NO:4); or GW NECASSNGHC SHLCLAVPVG GFVCGCPAHY SLNADNRTC (SEQ ID NO:5).

In another aspect, the invention provides compositions for eliciting antibodies that specifically bind to LRP6 when the composition is administered to an animal. The compositions include, for example, one of the following peptides: the first propeller of LRP6 (SEQ ID NO:2); NATNPCGIDN GGCSHLCLMS PVKPFYQCAC PTGVKLLENG KTCK (SEQ ID NO:3); the third propeller of LRP6 (SEQ ID NO:4); GW NECASSNGHC SHLCLAVPVG GFVCGCPAHY SLNADNRTC (SEQ ID NO:5); a peptide thereof with less than 5 amino acid changes; or a fragment thereof (e.g., fragments containing 5, 6, 7, 8, 9, 10, 11, or 12 amino acids). The peptide can be modified to increase antigenicity, e.g., by coupling to a carrier protein.

The invention also features a pharmaceutical composition that includes an LRP6 binding molecule described herein. The composition includes, for example, an LRP6 binding molecule and a pharmaceutically acceptable carrier.

The invention also features methods of using the LRP6 binding molecules described herein.

In one aspect, the invention features a method of inhibiting growth of a tumor cell. The method includes contacting the tumor cell with an antagonizing LRP6 binding molecule (e.g., an LRP6 binding molecule including an antigen binding portion of an antibody that specifically binds to an LRP6), thereby stabilizing the β-catenin destruction complex, engendering β-catenin phosphorylation and degradation, and preventing Wnt pathway signal transduction within the tumor cell.

In another aspect, the invention features a method of inducing apoptosis in a tumor cell. The method includes contacting the tumor cell, or the host cell environment, with an antagonizing LRP6 binding molecule (e.g., an LRP6 binding molecule including, an antigen binding portion of an antibody that specifically binds to an LRP6), thereby stabilizing the β-catenin destruction complex, engendering β-catenin phosphorylation and degradation, and preventing Wnt pathway signal transduction within the tumor cell.

In yet another aspect, the invention features a method of eroding stromal support for cancerous tumors. The method includes contacting the tumor cell with an antagonizing LRP6 binding molecule (e.g., an LRP6 binding molecule, including an antigen binding portion of an antibody that specifically binds to an LRP6), thereby killing blood vessels (angiogenic or otherwise) and/or other structural components on which the tumor relies.

In the above instances, the antagonizing LRP6 binding molecule can be administered in an amount effective to inhibit the growth of a tumor cell, or to induce apoptosis in a tumor cell, respectively. The subject's ratio of cancerous cells to healthy cells can be reduced by at least 10%, relative to said ratio prior to administering the composition (e.g., the ratio of cancerous cells to healthy cells is reduced by at least 25%, 30%, 50%, 60%, 75%, or 100%). In some embodiments, the subject is also receiving therapy with a chemotherapeutic agent.

In one aspect, the LRP6 agonizing or antagonizing binding molecules of the invention can be used to e.g., diagnose, ameliorate the symptoms of, protect against, and treat bone-related disorders, as defined herein.

In one aspect, the invention features a method of raising bone mineral density in a subject. The method includes administering an agonizing LRP6 binding molecule (e.g., an LRP6 binding molecule including an antigen binding portion of an antibody that specifically binds to an LRP6) to a subject in an amount effective to raise bone mineral density. In some embodiments, said agonizing LRP6 binding molecule interferes with binding events between LRP6 and Sclerostin and is capable of activating, enhancing, or perpetuating the Wnt signaling pathway, or sensitizing cells to Wnt signaling. Sclerostin is a protein encoded by the SOST gene, and is a negative regulator of the Wnt signaling pathway that is implicated in bone-related disorders (Wnt signaling is critical to bone formation under normal physiological conditions).

By way of non-limiting example, agonizing LRP6 binding molecules are capable of binding to LRP6 (e.g., to the 3rd propeller of LRP6) and engendering the dissolution of the β-catenin destruction complex, thereby permitting β-catenin stabilization, translocation to the nucleus, and engagement of transcription factors, thereby raising bone mineral density in the process. By way of other non-limiting example, agonizing LRP6 binding molecules are capable of binding to LRP6 elsewhere than the 3rd propeller. In these instances, said LRP6 agonist antibodies are capable of oligomerizing LRP6 such that Wnt signaling is activated, enhanced, or perpetuated, or that cells are sensitized to Wnt signaling

In another aspect, the invention features a method of lowering bone mineral density in a subject. The method includes administering an antagonizing LRP6 binding molecule (e.g., an LRP6 binding molecule including an antigen binding portion of an antibody that specifically binds to an LRP6) to a subject in an amount effective to lower bone mineral density. In some embodiments, said antagonizing LRP6 binding molecule mimics the effects of Sclerostin, and is thereby capable of negatively regulating the Wnt pathway in an analogous fashion.

In some embodiments, the antagonizing LRP6 binding molecules are Wnt1 specific, and bind to the first propeller of LRP6. In some other embodiments the antagonizing LRP6 binding molecules are Wnt1 specific, bind to the first propeller of LRP6, and prevent the ligands Wnt1, Wnt6, and/or Wnt7 from interacting with LRP6 and initiating the Wnt pathway.

In another aspect, the invention features a method of ameliorating or preventing symptoms of metabolic syndrome and/or coronary artery disease. The method includes administering an agonizing LRP6 binding molecule (e.g., an LRP6 binding molecule including an antigen binding portion of an antibody that specifically binds to an LRP6) to a subject in an amount effective to ameliorate or prevent symptoms of metabolic syndrome and/or coronary artery disease. In some embodiments, said agonizing LRP6 binding is capable of activating, enhancing, or perpetuating the Wnt signaling pathway, or sensitizing cells to Wnt signaling. By way of non-limiting example, said agonizing LRP6 binding molecules are capable of binding to LRP6 (e.g., to the 3rd propeller of LRP6), inducing oligomerization of LRP6, and engendering the dissolution of the β-catenin destruction complex, thereby permitting β-catenin stabilization, translocation to the nucleus, and engagement of transcription factors, thereby ameliorating or preventing symptoms of metabolic syndrome and/or coronary artery disease in the process.

In some embodiments, the composition is administered intravenously.

The details of one or more embodiments of the invention are set forth in the accompanying drawings and the description below. Other features, objects, and advantages of the invention will be apparent from the description and drawing, and from the claims.

### BRIEF DESCRIPTION OF DRAWINGS

The patent or application file contains at least one drawing executed in color. Copies of this patent or patent application publication with color drawing(s) will be provided by the Office upon request and payment of the necessary fee.
**Figure 1** is a graphical depiction of the identification of anti-LRP6 antagonistic Fabs which preferentially inhibit Wnt1 induced Wnt signaling.
**Figure 2** is a graphical depiction of the identification of anti-LRP6 antagonistic Fabs which preferentially inhibit Wnt3A induced Wnt signaling.
**Figure 3** is a graphical depiction of the identification of anti-LRP6 agonistic Fabs.
**Figure 4A** illustrates where LRP6 truncation mutants were generated, for LRP6 domain mapping. **Figure 4B** shows FACS analysis of the truncation mutants, with each graph corresponding to a different mutant. The top row, from left to right, features LRP6 full length, LRP6 deletion I, and LRP6 deletion II. The bottom row, from left to right, features LRP6 deletion III, LRP deletion I and II, and LRP6 deletion I and III. The cells were stained with anti-Flag antibody. **Figure 4C** shows FACS analysis of the truncation mutants, with each graph corresponding to a different mutant. The arrangement is the same as in Figure 4B, and the cells were stained with Fab005, Fab021, Fab010, Fab004, Fab002, Fab026, Fab025, and a control (anti-lysosome Fab).
**Figure 5** is a graphical depiction of the change in activity when a number of the Fabs are converted to IgGs. **Figure 5A** shows induction by Wnt1, and **Figure 5B** shows induction by Wnt3A.
**Figure 6** is a graphical demonstration of anti-LRP6 binding molecules' ability to inhibit phosphorylated LRP6 (phospho-LRP6). **Figure 6A** shows phospho-LRP6 inhibition in PA-1 (ovarian, teratocarcinoma) cells, and **Figure 6B** shows phospho-LRP6 inhibition in NCI-H929 (multiple myeloma) cells.

### DETAILED DESCRIPTION OF THE INVENTION

The present invention provides molecules that bind to LRP6 (low-density-lipoprotein receptor-related protein 6)("LRP6 binding molecules"), particularly human antibodies and portions thereof that bind to human LRP6 and modulate its functions. Epitopes of LRP6 and agents that bind these epitopes are also provided herein.

The full length sequence of human LRP6 (hLRP6) is found under Genbank® Accession Number GI:148727288, gb| NP_002327, and is shown in Table I as SEQ ID NO: 1. An mRNA sequence encoding hLRP6 is found under Accession Number GI: 148727287, NM_002336.

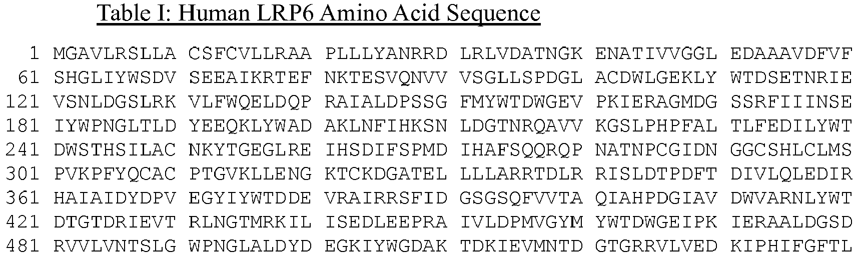

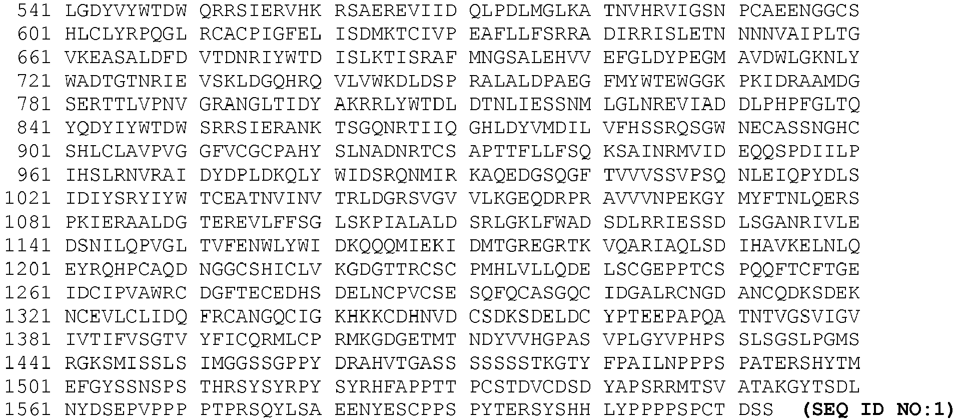

The locations of domains within the human LRP6 protein sequence (SEQ ID NO:1) is as follows: the signal peptide can be found from amino acid residues 1-19 of SEQ ID NO:1; the four YWTD (tyrosine, tryptophan, threonine, and aspartic acid)-type β-propeller domains, each of which forms a six-bladed β-propeller structure (Springer (1998) J. Mol. Biol.; 283:837; Jeon et al. (2001) Nat. Struct. Biol. 22:1172) can be found at amino acids 20-326 of SEQ ID NO:1; amino acids 327-630 of SEQ ID NO:1; amino acids 631-932 of SEQ ID NO:1; and amino acids 933-1246 of SEQ ID NO:1. Human LRP6 is N-glycosylated at N42, N81, N281, N433, N486, N692, N859, N865, N926, and N1039.

The amino acid sequence of mouse LRP6 has GenBank Accession No. GI: 148727327, NP_032540. The Drosphila Arrow sequence is found under Accession No. GI: 24653390, NP_524737, and the Xenopus LRP6 sequence is found under Accession No. GI: 10280605, AAG15429. Human LRP6 was identified by its homology to LRP5, which was originally isolated through its homology to LDLR (low-density-lipoprotein receptor). Arrow/LRP5/LRP6 are type I single-span transmembrane proteins with 1678, 1615 and 1613 amino acid residues, respectively. LRP5 and LRP6 share 73% and 64% identity in extracellular and intracellular domains, respectively, whereas Arrow is equally related (40% identical) to LRP5 and LRP6. Indeed, LRP6 substitutes for Arrow during Wg signaling in cultured *Drosophila* cells (Schweizer et al. (2003) BMC Cell Biol. 4, 4), and constitutively activated Arrow activates Wnt/β-catenin signaling in mammalian cells and Xenopus embryos (Tamai et al. (2004) Nature 407: 530).

### Definitions

As used herein, the term "Wnt signaling-related disorders" means diseases and conditions associated with aberrant Wnt signaling, including but not limited to cancers (e.g., colorectal carcinomas (CRCs), melanoma, breast, liver, lung, and gastric cancer; other, non-oncogenic proliferative diseases, such as proliferative skin disorders (e.g., psoriasis, dermatitis); osteoporosis; osteoarthritis; fibrotic disorders; schizophrenia; vascular disease; cardiac disease; aberrant hair growth or difficulty growing hair; wound healing; regenerative needs (liver, lung, limb); and neurodegenerative diseases such as Alzheimer's disease. Aberrant upregulation of Wnt signaling is associated with cancer, osteoarthritis, and polycystic kidney disease, while aberrant downregulation of Wnt signaling has been linked to osteoporosis, obesity, diabetes, and neuronal degenerative diseases.

As used herein, "Wnt signaling -related cancers" include but are not limited to colorectal carcinomas (CRCs), melanoma, breast, liver, lung, and gastric cancer. The term "Wnt-related cancer," as used herein also includes malignant medulloblastoma and other primary CNS malignant neuroectodermal tumors, rhabdomyosarcoma, lung cancer, gut-derived tumors, including but not limited to cancer of the esophagus, stomach, pancreas, and biliary duct system; prostate and bladder cancers; colon cancer; leukemias and other blood cancers (e.g., Chronic Lymphocytic Leukemia (CLL), Acute Myelogenous Leukemia (AML), Acute Lymphocytic Leukemia (ALL), Chronic Myelogenous Leukemia (CML), and Myelodysplastic Syndromes (MDS)); and lymphomas (e.g., Hodgkin lymphoma and non-Hodgkin lymphoma).

As used herein, "modulate" indicates the ability to control or influence directly or indirectly, and by way of non-limiting examples, can alternatively mean inhibit or stimulate, agonize or antagonize, hinder or promote, and strengthen or weaken.

As used herein, "antagonize" indicates the ability to inhibit or arrest, e.g., a signaling pathway such as Wnt. By way of example, an antagonizing LRP6 binding molecule of the invention can prevent signal transduction via the Wnt signaling pathway, by e.g., interfering with LRP6's ability to bind Wnt pathway members (e.g., DKK1 (dickkopf 1), DKK2, DKK4, SOST1, SOSD1 (USAG1), sFRP (soluble Fzd-related protein) 1-4, Wise, or Wnt ligands), and thereby promoting phosphorylation and degradation of β-catenin.

As used herein, "agonize" indicates the ability to engender, promote, enhance, or facilitate, e.g., a signaling pathway such as Wnt. By way of example, an agonizing LRP6 binding molecule of the invention can promoter or enhance signal transduction via the Wnt signaling pathway, by e.g., facilitating LRP6's ability to bind Wnt pathway members (e.g., DKK1 (dickkopf 1), DKK2, DKK4, SOST1, SOSD1 (USAG1), sFRP (soluble Fzd-related protein) 1-4, Wise, or Wnt ligands), and thereby preventing phosphorylation and degradation of β-catenin (thereby allowing β-catenin to reach the cell nucleus and bind transcription factors).

As used herein, "bone-related disorder" includes disorders in which bone mineral density (BMD) is abnormally and/or pathologically high relative to healthy subjects, and disorders in which bone mineral density (BMD) is abnormally and/or pathologically low relative to healthy subjects. Disorders characterized by high BMD include but are not limited to sclerosteosis, Van Buchem disease, bone overgrowth disorders, and Simpson-Golabi-Behmel syndrome (SGBS). Disorders characterized by low BMD and/or bone fragility include but are not limited to primary and secondary osteoporosis, osteopenia, osteomalacia, osteroporosis-pseudoglioma syndrome (OPPG), osteogenesis imperfecta (OI), avascular necrosis (osteonecrosis), fractures and implant healing (dental implants and hip implants), and bone loss due to other disorders (e.g., associated with HIV infection, cancers, or arthritis). Other "bone-related disorders" include but are not limited to rheumatoid arthritis, osteoarthritis, fracture, arthritis, and the formation and/or presence of osteolytic lesions.

As used herein, "fibrotic disorders" means any fibrotic disorder in humans characterized by excessive fibroblast or myofibroblast proliferation and production of connective tissue matrix, including collagen, fibronectin and glycosaminoglycans (GAG). Said disorders include but are not limited to: cutaneous keloid formation; progressive systemic sclerosis (PSS); liver cirrhosis; idiopathic and pharmacologically induced pulmonary fibrosis; chronic graft versus-host disease; scleroderma (local and systemic); Peyronie's disease; post-cystoscopic urethral stenosis; post-surgical internal adhesions; idiopathic and pharmacologically induced retroperitoneal fibrosis; and myelofibrosis.

As used herein, "fibrotic disorders" also includes but is not limited to lung fibrotic disorders (e.g., radiation-induced fibrosis, and fibrosis associated with asthma, COPD, and sarcoidosis); liver fibrotic disorders (e.g., alcoholic, Hepatitis C-associated, and primary biliary fibrosis, as well as non-alcoholic steatosis, sclerosing cholangitis, and fibrosis resulting from schistosomiasis); kidney fibrotic disorders (e.g., diabetic nephropathy, lupus glomerulosclerosis, Alport syndrome, and chronic renal allograft rejection); cardiovascular fibrotic disorders (e.g., post myocardial infarction scarring, cardiac hypertrophy, arterial restenosis, and atherosclerosis); skin fibrotic disorders (e.g., hypertrophic scarring, burn scarring, and nephrogenic fibrosing dermatopathy); eye fibrotic disorders (e.g., vitreoretinopathy and retroorbital fibrosis)

A "therapeutically effective dosage" of LRP6 binding molecule of the invention can results in a decrease in severity of disease symptoms (e.g., a decrease in symptoms of disorders associated with aberrantly high Wnt signaling (e.g., decrease in number, growth rate, or malignancy of tumor cells), or in symptoms of disorders associated with aberrantly low Wnt signaling (e.g., increase in LDL and triglyceride levels)), an increase in frequency and duration of disease symptom-free periods, or a prevention of impairment or disability due to the disease affliction.

The term "subject" is intended to include organisms, e.g., eukaryotes, which are suffering from or afflicted with a disease, disorder or condition associated with aberrant Wnt signaling. Examples of subjects include mammals, e.g., humans, dogs, cows, horses, pigs, sheep, goats, cats, mice, rabbits, rats, and transgenic non-human animals. In certain embodiments, the subject is a human, e.g., a human suffering from, at risk of suffering from, or potentially capable of suffering from cancer (e.g., colon cancer) and other proliferative diseases, osteoporosis, and schizophrenia, and other diseases or conditions described herein (e.g., a Wnt signaling-related disorder).

The term "antibody" as used herein refers to an intact antibody or an antigen binding fragment (i.e., "antigen-binding portion") or single chain (i.e., light or heavy chain) thereof. An intact antibody is a glycoprotein comprising at least two heavy (H) chains and two light (L) chains inter-connected by disulfide bonds. Each heavy chain is comprised of a heavy chain variable region (abbreviated herein as V_{H}) and a heavy chain constant region. The heavy chain constant region is comprised of three domains, CH1, CH2 and CH3. Each light chain is comprised of a light chain variable region (abbreviated herein as V_{L}) and a light chain constant region. The light chain constant region is comprised of one domain, C_{L}. The V_{H} and V_{L} regions can be further subdivided into regions of hypervariability, termed complementarity determining regions (CDR), interspersed with regions that are more conserved, termed framework regions (FR). Each V_{H} and V_{L} is composed of three CDRs and four FRs arranged from amino-terminus to carboxy-terminus in the following order: FR1, CDR1, FR2, CDR2, FR3, CDR3, FR4. The variable regions of the heavy and light chains contain a binding domain that interacts with an antigen. The constant regions of the antibodies may mediate the binding of the immunoglobulin to host tissues or factors, including various cells of the immune system (e.g., effector cells) and the first component (Clq) of the classical complement system.

The term "antigen binding portion" of an antibody, as used herein, refers to one or more fragments of an intact antibody that retain the ability to specifically bind to a given antigen (e.g., hLRP6). Antigen binding functions of an antibody can be performed by fragments of an intact antibody. Examples of binding fragments encompassed within the term "antigen binding portion" of an antibody include a Fab fragment, a monovalent fragment consisting of the V_{L}, V_{H}, C_{L} and CH1 domains; an F(ab)₂ fragment, a bivalent fragment comprising two Fab fragments (generally one from a heavy chain and one from a light chain) linked by a disulfide bridge at the hinge region; an Fd fragment consisting of the V_{H} and CH1 domains; an Fv fragment consisting of the V_{L} and V_{H} domains of a single arm of an antibody; a single domain antibody (dAb) fragment (Ward et al., 1989 Nature 341:544-546), which consists of a V_{H} domain; and an isolated complementarity determining region (CDR).

Furthermore, although the two domains of the Fv fragment, V_{L} and V_{H}, are coded for by separate genes, they can be joined, using recombinant methods, by an artificial peptide linker that enables them to be made as a single protein chain in which the V_{L} and V_{H} regions pair to form monovalent molecules (known as single chain Fv (scFv); see, e.g., Bird et al., 1988 Science 242:423-426; and Huston et al., 1988 Proc. Natl. Acad. Sci. 85:5879-5883). Such single chain antibodies include one or more "antigen binding portions" of an antibody. These antibody fragments are obtained using conventional techniques known to those of skill in the art, and the fragments are screened for utility in the same manner as are intact antibodies.

Antigen binding portions can also be incorporated into single domain antibodies, maxibodies, minibodies, intrabodies, diabodies, triabodies, tetrabodies, v-NAR and bis-scFv (see, e.g., Hollinger and Hudson, 2005, Nature Biotechnology, 23, 9, 1126-1136). Antigen binding portions of antibodies can be grafted into scaffolds based on polypeptides such as Fibronectin type III (Fn3) (see U.S. Pat. No. 6,703,199, which describes fibronectin polypeptide monobodies).

Antigen binding portions can be incorporated into single chain molecules comprising a pair of tandem Fv segments (V_{H}-CH1-V_{H}-CH1) which, together with complementary light chain polypeptides, form a pair of antigen binding regions (Zapata et al., 1995 Protein Eng. 8(10):1057-1062; and U.S. Pat. No. 5,641,870).

The term "camelid antibody," as used herein, refers to one or more fragments of an intact antibody protein obtained from members of the camel and dromedary (Camelus bactrianus and Calelus dromaderius) family, including New World members such as llama species (Lama paccos, Lama glama and Lama vicugna). A region of the camelid antibody that is the small, single variable domain identified as V_{HH} can be obtained by genetic engineering to yield a small protein having high affinity for a target, resulting in a low molecular weight, antibody-derived protein known as a "camelid nanobody". See U.S. Pat. No. 5,759,808; see also Stijlemans et al., 2004 J. Biol. Chem. 279: 1256-1261; Dumoulin et al., 2003 Nature 424: 783-788; Pleschberger et al., 2003 Bioconjugate Chem. 14: 440-448; Cortez-Retamozo et al., 2002 Int. J. Cancer 89: 456-62; and Lauwereys. et al., 1998 EMBO J. 17: 3512-3520.

An "isolated LRP6 binding molecule", as used herein, refers to a binding molecule that is substantially free of molecules having antigenic specificities for antigens other than LRP6 (e.g., an isolated antibody that specifically binds hLRP6 is substantially free of antibodies that specifically bind antigens other than hLRP6). An isolated binding molecule that specifically binds hLRP6 may, however, have cross-reactivity to other antigens, such as LRP6 molecules from other species. A binding molecule is "purified" if it is substantially free of cellular material.

The term "monoclonal antibody composition" as used herein refers to a preparation of antibody molecules of single molecular composition. A monoclonal antibody composition displays a single binding specificity and affinity for a particular epitope.

The term "human antibody," as used herein, is intended to include antibodies having variable regions in which both the framework and CDR regions are derived from sequences of human origin. Furthermore, if the antibody contains a constant region, the constant region also is derived from such human sequences, e.g., human germline sequences, or mutated versions of human germline sequences. The human antibodies of the invention may include amino acid residues not encoded by human sequences (e.g., mutations introduced by random or site-specific mutagenesis in vitro or by somatic mutation in vivo). However, the term "human antibody", as used herein, is not intended to include antibodies in which CDR sequences derived from the germline of another mammalian species, such as a mouse, have been grafted onto human framework sequences.

The term "human monoclonal antibody" refers to an antibody displaying a single binding specificity that has variable regions in which both the framework and CDR regions are derived from human sequences. In one embodiment, the human monoclonal antibody is produced by a hybridoma that includes a B cell obtained from a transgenic nonhuman animal (e.g., a transgenic mouse having a genome comprising a human heavy chain transgene and a light chain transgene) fused to an immortalized cell.

The term "recombinant human antibody", as used herein, includes any human antibody that is prepared, expressed, created or isolated by recombinant means, such as an antibody isolated from an animal (e.g., a mouse) that is transgenic or transchromosomal for human immunoglobulin genes or a hybridoma prepared therefrom; an antibody isolated from a host cell transformed to express the human antibody, e.g., from a transfectoma; an antibody isolated from a recombinant, combinatorial human antibody library; and an antibody prepared, expressed, created or isolated by any other means that involve splicing of all or a portion of a human immunoglobulin gene sequences to another DNA sequence. Such recombinant human antibodies have variable regions in which the framework and CDR regions are derived from human germline immunoglobulin sequences. In certain embodiments, however, such recombinant human antibodies can be subjected to in vitro mutagenesis (or, when an animal transgenic for human Ig sequences is used, in vivo somatic mutagenesis) and thus the amino acid sequences of the V_{H} and V_{L} regions of the recombinant antibodies are sequences that, while derived from and related to human germline V_{H} and V_{L} sequences, may not naturally exist within the human antibody germline repertoire in a human.

As used herein, "isotype" refers to the antibody class (e.g., IgM, IgE, IgG such as IgG1 or IgG4) that is encoded by the heavy chain constant region gene.

The phrases "an antibody recognizing an antigen" and "an antibody specific for an antigen" are used interchangeably herein with the term "an antibody that binds specifically to an antigen."

As used herein, an LRP6 binding molecule (e.g., an antibody or antigen binding portion thereof) that "specifically binds to LRP6 " is intended to refer to an LRP6 binding molecule that binds to LRP6 with a K_{D} of 1 x 10⁻⁷ M or less. A LRP6 binding molecule (e.g., an antibody) that "cross-reacts with an antigen" is intended to refer to an LRP6 binding molecule that binds that antigen with a K_{D} of 1 x 10⁻⁶ M or less. A LRP6 binding molecule (e.g., an antibody) that "does not cross-react" with a given antigen is intended to refer to an LRP6 binding molecule that either does not bind detectably to the given antigen, or binds with a K_{D} of 1 x 10⁻⁵ M or greater. In certain embodiments, such binding molecules that do not cross-react with the antigen exhibit essentially undetectable binding against these proteins in standard binding assays.

As used herein, the term "high affinity", when referring to an IgG antibody, indicates that the antibody has a K_{D} of 10⁻⁹ M or less for a target antigen.

A nucleotide sequence is said to be "optimized" if it has been altered to encode an amino acid sequence using codons that are preferred in the production cell or organism, generally a eukaryotic cell, for example, a cell of a yeast such as *Pichia,* an insect cell, a mammalian cell such as Chinese Hamster Ovary cell (CHO) or a human cell. The optimized nucleotide sequence is engineered to encode an amino acid sequence identical or nearly identical to the amino acid sequence encoded by the original starting nucleotide sequence, which is also known as the "parental" sequence.

Various aspects of the invention are described in further detail in the following subsections.

Standard assays to evaluate the ability of molecules to bind to LRP6 of various species, and particular epitopes of LRP6, are known in the art, including, for example, ELISAs and western blots. Determination of whether an LRP6 binding molecule binds to a specific epitope of LRP6 can employ a peptide epitope competition assay. For example, an LRP6 binding molecule is incubated with a peptide corresponding to an LRP6 epitope of interest at saturating concentrations of peptide. The preincubated LRP6 binding molecule is tested for binding to immobilized LRP6, e.g., by Biacore® analysis. Inhibition of LRP6 binding by preincubation with the peptide indicates that the LRP6 binding molecule binds to the peptide epitope (see, e.g., U.S. Pat. Pub. 20070072797). Binding kinetics also can be assessed by standard assays known in the art, such as by Biacore® analysis or apparent binding by FACS analysis. Assays to evaluate the effects of LRP6 binding molecules on functional properties of LRP6 are described in further detail below.

Accordingly, an LRP6 binding molecule that "inhibits" one or more of these LRP6 functional properties (e.g., biochemical, cellular, physiological or other biological activities, or the like), as determined according to methodologies known to the art and described herein, will be understood to produce a statistically significant decrease in the particular functional property relative to that seen in the absence of the binding molecule (e.g., when a control molecule of irrelevant specificity is present). A LRP6 binding molecule that inhibits LRP6 activity effects such a statistically significant decrease by at least 5% of the measured parameter. In certain embodiments, an antagonizing antibody or other LRP6 binding molecule may produce a decrease in the selected functional property of at least 10%, 20%, 30%, or 50% compared to control.

In some embodiments, LRP6 inhibition is determined by measuring levels of proteins or protein stability in the Wnt signaling pathway (e.g., Wnt, Wise, DKK1, DKK2, DKK4, SOST1, SOSD1 (USAG1), sFRP (soluble Fzd-related protein) 1-4, Axin, or β-catenin). In other embodiments, biological, physiological, and/or morphological changes indicate that the LRP6 binding molecule inhibits LRP6, e.g., to inhibit the growth of a tumor cell or to induce apoptosis in a tumor cell; or to lower bone mineral density.

Contrarily, a LRP6 binding molecule that agonizes or promotes LRP6 activity effectuates such a statistically significant increase by at least 5% of the measured parameter. In certain embodiments, an agonizing antibody or other LRP6 binding molecule may produce a increase in the selected functional property of at least 10%, 20%, 30%, or 50% compared to control.

In some embodiments, LRP6 inhibition is determined by measuring expression or stability levels of downstream mRNA messages or proteins in the Wnt signaling pathway (e.g., Axin, Axin2, β-catenin, VEGF, cMyc, cyclin D1, SNAIL). In other embodiments, biological, physiological, and/or morphological changes indicate that the LRP6 binding molecule inhibits Wnt signaling, e.g., lower than normal bone mineral density or insulin secretion.

### Antibodies

The anti-LRP6 antibodies described herein include human monoclonal antibodies. In some embodiments, antigen binding portions of antibodies that bind to LRP6, (e.g., V_{H} and V_{L} chains) are "mixed and matched" to create other anti-LRP6 binding molecules. The binding of such "mixed and matched" antibodies can be tested using the aforementioned binding assays (e.g., FACS, ELISAs). When selecting a V_{H} to mix and match with a particular V_{L} sequence, typically one selects a V_{H} that is structurally similar to the V_{H} it replaces in the pairing with that V_{L}. Likewise a full length heavy chain sequence from a particular full length heavy chain/full length light chain pairing is generally replaced with a structurally similar full length heavy chain sequence. Likewise, a V_{L} sequence from a particular V_{H}/V_{L} pairing should be replaced with a structurally similar V_{L} sequence. Likewise a full length light chain sequence from a particular full length heavy chain/full length light chain pairing should be replaced with a structurally similar full length light chain sequence. Identifying structural similarity in this context is a process well known in the art.

In other aspects, the invention provides antibodies that comprise the heavy chain and light chain CDRls, CDR2s and CDR3s of one or more LRP6-binding antibodies, in various combinations. Given that each of these antibodies can bind to LRP6 and that antigen-binding specificity is provided primarily by the CDR1, 2 and 3 regions, the V_{H} CDR1, 2 and 3 sequences and V_{L} CDR1, 2 and 3 sequences can be "mixed and matched" (i.e., CDRs from different antibodies can be mixed and matched). LRP6 binding of such "mixed and matched" antibodies can be tested using the binding assays described herein (e.g., ELISAs). When V_{H} CDR sequences are mixed and matched, the CDR1, CDR2 and/or CDR3 sequence from a particular V_{H} sequence should be replaced with a structurally similar CDR sequence(s). Likewise, when V_{L} CDR sequences are mixed and matched, the CDR1, CDR2 and/or CDR3 sequence from a particular V_{L} sequence should be replaced with a structurally similar CDR sequence(s). Identifying structural similarity in this context is a process well known in the art.

As used herein, a human antibody comprises heavy or light chain variable regions or full length heavy or light chains that are "the product of" or "derived from" a particular germline sequence if the variable regions or full length chains of the antibody are obtained from a system that uses human germline immunoglobulin genes as the source of the sequences. In one such system, a human antibody is raised in a transgenic mouse carrying human immunoglobulin genes. The transgenic is immunized with the antigen of interest (e.g., an epitope of hLRP6 described herein). Alternatively, a human antibody is identified by providing a human immunoglobulin gene library displayed on phage and screening the library with the antigen of interest (e.g., hLRP6 or an hLRP6 epitope described herein).

A human antibody that is "the product of" or "derived from" a human germline immunoglobulin sequence can be identified as such by comparing the amino acid sequence of the human antibody to the amino acid sequences of human germline immunoglobulins and selecting the human germline immunoglobulin sequence that is closest in sequence (i.e., greatest % identity) to the sequence of the human antibody. A human antibody that is "the product of" or "derived from" a particular human germline immunoglobulin sequence may contain amino acid differences as compared to the germline-encoded sequence, due to, for example, naturally occurring somatic mutations or artificial site-directed mutations. However, a selected human antibody typically has an amino acid sequence at least 90% identical to an amino acid sequence encoded by a human germline immunoglobulin gene and contains amino acid residues that identify the human antibody as being human when compared to the germline immunoglobulin amino acid sequences of other species (e.g., murine germline sequences). In certain cases, a human antibody may be at least 60%, 70%, 80%, 90%, or at least 95%, or even at least 96%, 97%, 98%, or 99% identical in amino acid sequence to the amino acid sequence encoded by the germline immunoglobulin gene.

The percent identity between two sequences is a function of the number of identity positions shared by the sequences (i.e., % identity = # of identity positions/total # of positions x 100), taking into account the number of gaps, and the length of each gap, that need to be introduced for optimal alignment of the two sequences. The comparison of sequences and determination of percent identity between two sequences is determined using the algorithm of E. Meyers and W. Miller (1988 Comput. Appl. Biosci., 4:11-17) which has been incorporated into the ALIGN program (version 2.0), using a PAM120 weight residue table, a gap length penalty of 12 and a gap penalty of 4.

Typically, a V_{H} or V_{L} of a human antibody derived from a particular human germline sequence will display no more than 10 amino acid differences from the amino acid sequence encoded by the human germline immunoglobulin gene. In certain cases, the V_{H} or V_{L} of the human antibody may display no more than 5, or even no more than 4, 3, 2, or 1 amino acid difference from the amino acid sequence encoded by the germline immunoglobulin gene.

### Camelid antibodies

Antibody proteins obtained from members of the camel and dromedary (*Camelus bactrianus* and *Calelus dromaderius*) family, including New World members such as llama species (*Lama paccos, Lama glama and Lama vicugna)*, have been characterized with respect to size, structural complexity and antigenicity for human subjects. Certain IgG antibodies found in nature in this family of mammals lack light chains, and are thus structurally distinct from the four chain quaternary structure having two heavy and two light chains typical for antibodies from other animals. See WO 94/04678.

A region of the camelid antibody that is the small, single variable domain identified as V_{HH} can be obtained by genetic engineering to yield a small protein having high affinity for a target, resulting in a low molecular weight, antibody-derived protein known as a "camelid nanobody". See U.S. Pat. No. 5,759,808; see also Stijlemans et al., 2004 J. Biol. Chem. 279: 1256-1261; Dumoulin et al., 2003 Nature 424: 783-788; Pleschberger et al., 2003 Bioconjugate Chem. 14: 440-448; Cortez-Retamozo et al., 2002 Int. J. Cancer 89: 456-62; and Lauwereys. et al., 1998 EMBO J. 17: 3512-3520. Engineered libraries of camelid antibodies and antibody fragments are commercially available, for example, from Ablynx, Ghent, Belgium. As with other antibodies of non-human origin, an amino acid sequence of a camelid antibody can be altered recombinantly to obtain a sequence that more closely resembles a human sequence, i.e., the nanobody can be "humanized". Thus the natural low antigenicity of camelid antibodies to humans can be further reduced.

The camelid nanobody has a molecular weight approximately one-tenth that of a human IgG molecule, and the protein has a physical diameter of only a few nanometers. One consequence of the small size is the ability of camelid nanobodies to bind to antigenic sites that are functionally invisible to larger antibody proteins, i.e., camelid nanobodies are useful as reagents to detect antigens that are otherwise cryptic using classical immunological techniques, and as possible therapeutic agents. Thus, yet another consequence of small size is that a camelid nanobody can inhibit as a result of binding to a specific site in a groove or narrow cleft of a target protein, and hence can serve in a capacity that more closely resembles the function of a classical low molecular weight drug than that of a classical antibody.

The low molecular weight and compact size further result in camelid nanobodies' being extremely thermostable, stable to extreme pH and to proteolytic digestion, and poorly antigenic. Another consequence is that camelid nanobodies readily move from the circulatory system into tissues, and even cross the blood-brain barrier and can treat disorders that affect nervous tissue. Nanobodies can further facilitate drug transport across the blood brain barrier. See U.S. Pat. Pub. No. 20040161738, published August 19, 2004. These features combined with the low antigenicity in humans indicate great therapeutic potential. Further, these molecules can be fully expressed in prokaryotic cells such as *E. coli.*

Accordingly, a feature of the present invention is a camelid antibody or camelid nanobody having high affinity for LRP6. In certain embodiments herein, the camelid antibody or nanobody is naturally produced in the camelid animal, i.e., is produced by the camelid following immunization with LRP6 or a peptide fragment thereof, using techniques described herein for other antibodies. Alternatively, the anti-LRP6 camelid nanobody is engineered, i.e., produced by selection, for example from a library of phage displaying appropriately mutagenized camelid nanobody proteins using panning procedures with LRP6 or an LRP6 epitope described herein as a target. Engineered nanobodies can further be customized by genetic engineering to increase the half life in a recipient subject from 45 minutes to two weeks.

### Diabodies

Diabodies are bivalent, bispecific molecules in which V_{H} and V_{L} domains are expressed on a single polypeptide chain, connected by a linker that is too short to allow for pairing between the two domains on the same chain. The V_{H} and V_{L} domains pair with complementary domains of another chain, thereby creating two antigen binding sites (see e.g., Holliger et al., 1993 Proc. Natl. Acad. Sci. USA 90:6444-6448; Poljak et al., 1994 Structure 2:1121-1123). Diabodies can be produced by expressing two polypeptide chains with either the structure V_{HA}-V_{LB} and V_{HB}-V_{LA} (V_{H}-V_{L} configuration), or V_{LA}-V_{HB} and V_{LB}-V_{HA} (V_{L}-V_{H} configuration) within the same cell. Most of them can be expressed in soluble form in bacteria.

Single chain diabodies (scDb) are produced by connecting the two diabody-forming polypeptide chains with linker of approximately 15 amino acid residues (see Holliger and Winter, 1997 Cancer Immunol. Immunother., 45(3-4):128-30; Wu et al., 1996 Immunotechnology, 2(1):21-36). scDb can be expressed in bacteria in soluble, active monomeric form (see Holliger and Winter, 1997 Cancer Immunol. Immunother., 45(34): 128-30; Wu et al., 1996 Immunotechnology, 2(1):21-36; Pluckthun and Pack, 1997 Immunotechnology, 3(2): 83-105; Ridgway et al., 1996 Protein Eng., 9(7):617-21).

A diabody can be fused to Fc to generate a "di-diabody" (see Lu et al., 2004 J. Biol. Chem., 279(4):2856-65).

### Engineered and modified antibodies

An antibody of the invention can be prepared using an antibody having one or more V_{H} and/or V_{L} sequences as starting material to engineer a modified antibody, which modified antibody may have altered properties from the starting antibody. An antibody can be engineered by modifying one or more residues within one or both variable regions (i. e., V_{H} and/or V_{L}), for example within one or more CDR regions and/or within one or more framework regions. Additionally or alternatively, an antibody can be engineered by modifying residues within the constant region(s), for example to alter the effector function(s) of the antibody.

One type of variable region engineering that can be performed is CDR grafting. Antibodies interact with target antigens predominantly through amino acid residues that are located in the six heavy and light chain CDRs. For this reason, the amino acid sequences within CDRs are more diverse between individual antibodies than sequences outside of CDRs. Because CDR sequences are responsible for most antibody-antigen interactions, it is possible to express recombinant antibodies that mimic the properties of specific naturally occurring antibodies by constructing expression vectors that include CDR sequences from the specific naturally occurring antibody grafted onto framework sequences from a different antibody with different properties (see, e.g., Riechmann et al., 1998 Nature 332:323-327; Jones et al., 1986 Nature 321:522-525; Queen et al., 1989 Proc. Natl. Acad. See. U.S.A. 86:10029-10033; U.S. Pat. No. 5,225,539, and U.S. Pat. Nos. 5,530,101; 5,585,089; 5,693,762 and 6,180,370).

Framework sequences can be obtained from public DNA databases or published references that include germline antibody gene sequences. For example, germline DNA sequences for human heavy and light chain variable region genes can be found in the "VBase" human germline sequence database (available on the Internet at www.mrc- cpe.cam.ac.uk/vbase), as well as in Kabat et al., 1991 Sequences of Proteins of Immunological Interest, Fifth Edition, U.S. Department of Health and Human Services, NIH Publication No. 91-3242; Tomlinson et al., 1992 J. Mol. Biol. 227:776-798; and Cox et al., 1994 Eur. J. Immunol. 24:827-836; the contents of each of which are expressly incorporated herein by reference.

The V_{H} CDR1, 2 and 3 sequences and the V_{L} CDR1, 2 and 3 sequences can be grafted onto framework regions that have the identical sequence as that found in the germline immunoglobulin gene from which the framework sequence is derived, or the CDR sequences can be grafted onto framework regions that contain one or more mutations as compared to the germline sequences. For example, it has been found that in certain instances it is beneficial to mutate residues within the framework regions to maintain or enhance the antigen binding ability of the antibody (see e.g., U.S. Pat. Nos. 5,530,101; 5,585,089; 5,693,762 and 6,180,370).

CDRs can also be grafted into framework regions of polypeptides other than immunoglobulin domains. Appropriate scaffolds form a conformationally stable framework that displays the grafted residues such that they form a localized surface and bind the target of interest (e.g., LRP6). For example, CDRs can be grafted onto a scaffold in which the framework regions are based on fibronectin, ankyrin, lipocalin, neocarzinostain, cytochrome b, CP1 zinc finger, PST1, coiled coil, LACI-D1, Z domain or tendramisat (See e.g., Nygren and Uhlen, 1997 Current Opinion in Structural Biology, 7, 463-469).

Another type of variable region modification is mutation of amino acid residues within the V_{H} and/or V_{L} CDR1, CDR2 and/or CDR3 regions to thereby improve one or more binding properties (e.g., affinity) of the antibody of interest, known as "affinity maturation." Site-directed mutagenesis or PCR-mediated mutagenesis can be performed to introduce the mutation(s), and the effect on antibody binding, or other functional property of interest, can be evaluated in in vitro or in vivo assays as described herein. Conservative modifications can be introduced. The mutations may be amino acid substitutions, additions or deletions. Moreover, typically no more than one, two, three, four or five residues within a CDR region are altered.

Engineered antibodies of the invention include those in which modifications have been made to framework residues within V_{H} and/or V_{L}, e.g., to improve the properties of the antibody. Typically such framework modifications are made to decrease the immunogenicity of the antibody. For example, one approach is to "backmutate" one or more framework residues to the corresponding germline sequence. More specifically, an antibody that has undergone somatic mutation may contain framework residues that differ from the germline sequence from which the antibody is derived. Such residues can be identified by comparing the antibody framework sequences to the germline sequences from which the antibody is derived. To return the framework region sequences to their germline configuration, the somatic mutations can be "backmutated" to the germline sequence by, for example, site-directed mutagenesis or PCR-mediated mutagenesis. Such "backmutated" antibodies are also intended to be encompassed by the invention.

Another type of framework modification involves mutating one or more residues within the framework region, or even within one or more CDR regions, to remove T cell - epitopes to thereby reduce the potential immunogenicity of the antibody. This approach is also referred to as "deimmunization" and is described in further detail in U.S. Pat. Pub. No. 20030153043 by Carr et al.

In addition or alternative to modifications made within the framework or CDR regions, antibodies of the invention may be engineered to include modifications within the Fc region, typically to alter one or more functional properties of the antibody, such as serum half-life, complement fixation, Fc receptor binding, and/or antigen-dependent cellular cytotoxicity. Furthermore, an antibody of the invention may be chemically modified (e.g., one or more chemical moieties can be attached to the antibody) or be modified to alter its glycosylation, again to alter one or more functional properties of the antibody.

In one embodiment, the hinge region of CH1 is modified such that the number of cysteine residues in the hinge region is altered, e.g., increased or decreased. This approach is described further in U.S. Pat. No. 5,677,425 by Bodmer et al. The number of cysteine residues in the hinge region of CH1 is altered to, for example, facilitate assembly of the light and heavy chains or to increase or decrease the stability of the antibody.

In another embodiment, the Fc hinge region of an antibody is mutated to decrease the biological half-life of the antibody. More specifically, one or more amino acid mutations are introduced into the CH2-CH3 domain interface region of the Fc-hinge fragment such that the antibody has impaired Staphylococcyl protein A (SpA) binding relative to native Fc-hinge domain SpA binding. This approach is described in further detail in U.S. Pat. No. 6,165,745 by Ward et al.

In another embodiment, the antibody is modified to increase its biological half-life. Various approaches are possible. For example, U.S. Pat. No. 6,277,375 describes the following mutations in an IgG that increase its half-life in vivo: T252L, T254S, T256F. Alternatively, to increase the biological half life, the antibody can be altered within the CH1 or CL region to contain a salvage receptor binding epitope taken from two loops of a CH2 domain of an Fc region of an IgG, as described in U.S. Pat. Nos. 5,869,046 and 6,121,022 by Presta et al.

In yet other embodiments, the Fc region is altered by replacing at least one amino acid residue with a different amino acid residue to alter the effector functions of the antibody. For example, one or more amino acids can be replaced with a different amino acid residue such that the antibody has an altered affinity for an effector ligand but retains the antigen-binding ability of the parent antibody. The effector ligand to which affinity is altered can be, for example, an Fc receptor or the C1 component of complement. This approach is described in further detail in U.S. Pat. Nos. 5,624,821 and 5,648,260, both by Winter et al.

In another embodiment, one or more amino acids selected from amino acid residues can be replaced with a different amino acid residue such that the antibody has altered C1q binding and/or reduced or abolished complement dependent cytotoxicity (CDC). This approach is described in further detail in U.S. Pat. Nos. 6,194,551 by Idusogie et al.

In another embodiment, one or more amino acid residues are altered to thereby alter the ability of the antibody to fix complement. This approach is described further in WO 94/29351 by Bodmer et al.

In yet another embodiment, the Fc region is modified to increase the ability of the antibody to mediate antibody dependent cellular cytotoxicity (ADCC) and/or to increase the affinity of the antibody for an Fcγ receptor by modifying one or more amino acids. This approach is described further in WO 00/42072 by Presta. Moreover, the binding sites on human IgG1 for FcγR1, FcγRII, FcγRIII and FcRn have been mapped and variants with improved binding have been described (see Shields, R.L. et al., 2001 J. Biol. Chem. 276:6591-6604).

In still another embodiment, the glycosylation of an antibody is modified. For example, an aglycoslated antibody can be made (i.e., the antibody lacks glycosylation). Glycosylation can be altered, for example, to increase the affinity of the antibody for an antigen. Such carbohydrate modifications can be accomplished by, for example, altering one or more sites of glycosylation within the antibody sequence. For example, one or more amino acid substitutions can be made that result in elimination of one or more variable region framework glycosylation sites to thereby eliminate glycosylation at that site. Such aglycosylation may increase the affinity of the antibody for antigen. Such an approach is described in further detail in U.S. Pat. Nos. 5,714,350 and 6,350,861 by Co et al.

Additionally or alternatively, an antibody can be made that has an altered type of glycosylation, such as a hypofucosylated antibody having reduced amounts of fucosyl residues or an antibody having increased bisecting GlcNac structures. Such altered glycosylation patterns have been demonstrated to increase the ADCC ability of antibodies. Such carbohydrate modifications can be accomplished by, for example, expressing the antibody in a host cell with altered glycosylation machinery. Cells with altered glycosylation machinery have been described in the art and can be used as host cells in which to express recombinant antibodies of the invention to thereby produce an antibody with altered glycosylation. For example, EP 1,176,195 by Hang et al. describes a cell line with a functionally disrupted FUT8 gene, which encodes a fucosyl transferase, such that antibodies expressed in such a cell line exhibit hypofucosylation. PCT Pub. WO 03/035835 by Presta describes a variant CHO cell line, Lecl3 cells, with reduced ability to attach fucose to Asn(297)-linked carbohydrates, also resulting in hypofucosylation of antibodies expressed in that host cell (see also Shields, R.L. et al., 2002 J. Biol. Chem. 277:26733-26740). WO 99/54342 by Umana et al. describes cell lines engineered to express glycoprotein-modifying glycosyl transferases (e.g., beta(1,4)-N acetylglucosaminyltransferase III (GnTIII)) such that antibodies expressed in the engineered cell lines exhibit increased bisecting GlcNac structures which results in increased ADCC activity of the antibodies (see also Umana et al., 1999 Nat. Biotech. 17:176-180).

Another modification of the antibodies herein that is contemplated by the invention is pegylation. An antibody can be pegylated to, for example, increase the biological (e.g., serum) half-life of the antibody. To pegylate an antibody, the antibody, or fragment thereof, typically is reacted with polyethylene glycol (PEG), such as a reactive ester or aldehyde derivative of PEG, under conditions in which one or more PEG moieties become attached to the antibody or antibody fragment. The pegylation can be carried out by an acylation reaction or an alkylation reaction with a reactive PEG molecule (or an analogous reactive water-soluble polymer). As used herein, the term "polyethylene glycol" is intended to encompass any of the forms of PEG that have been used to derivatize other proteins, such as mono (C1-C10) alkoxy- or aryloxy-polyethylene glycol or polyethylene glycol-maleimide. In certain embodiments, the antibody to be pegylated is an aglycosylated antibody. Methods for pegylating proteins are known in the art and can be applied to the antibodies of the invention. See for example, EP 0 154 316 by Nishimura et al. and EP 0 401 384 by Ishikawa et al.

In addition, pegylation can be achieved in any part of an LRP6 binding polypeptide of the invention by the introduction of a nonnatural amino acid. Certain nonnatural amino acids can be introduced by the technology described in Deiters et al., J Am Chem Soc 125:11782-11783, 2003; Wang and Schultz, Science 301:964-967, 2003; Wang et al., Science 292:498-500, 2001; Zhang et al., Science 303:371-373, 2004 or in US Patent No. 7,083,970. Briefly, some of these expression systems involve site-directed mutagenesis to introduce a nonsense codon, such as an amber TAG, into the open reading frame encoding a polypeptide of the invention. Such expression vectors are then introduced into a host that can utilize a tRNA specific for the introduced nonsense codon and charged with the nonnatural amino acid of choice. Particular nonnatural amino acids that are beneficial for purpose of conjugating moieties to the polypeptides of the invention include those with acetylene and azido side chains. The polypeptides containing these novel amino acids can then be pegylated at these chosen sites in the protein.

### Methods of engineering antibodies

As discussed above, anti-LRP6 antibodies can be used to create new anti-LRP6 antibodies by modifying full length heavy chain and/or light chain sequences, V_{H} and/or V_{L} sequences, or the constant region(s) attached thereto. For example, one or more CDR regions of the antibodies can be combined recombinantly with known framework regions and/or other CDRs to create new, recombinantly-engineered, anti-LRP6 antibodies. Other types of modifications include those described in the previous section. The starting material for the engineering method is one or more of the V_{H} and/or V_{L} sequences, or one or more CDR regions thereof. To create the engineered antibody, it is not necessary to actually prepare (i.e., express as a protein) an antibody having one or more of the V_{H} and/or V_{L} sequences, or one or more CDR regions thereof. Rather, the information contained in the sequence(s) is used as the starting material to create a "second generation" sequence(s) derived from the original sequence(s) and then the "second generation" sequence(s) is prepared and expressed as a protein.

Standard molecular biology techniques can be used to prepare and express the altered antibody sequence. The antibody encoded by the altered antibody sequence(s) is one that retains one, some or all of the functional properties of the anti-LRP6 antibody from which it is derived, which functional properties include, but are not limited to, specifically binding to LRP6, interfering with LRP6's ability to bind Wnt pathway members (e.g., DKK1 (dickkopf 1), DKK2, DKK4, SOST1, SOSD1 (USAG1), sFRP (soluble Fzd-related protein) 1-4, Wise, or Wnt ligands), and modulating β-catenin phosphorylation and degradation. The functional properties of the altered antibodies can be assessed using standard assays available in the art and/or described herein (e.g., ELISAs).

In certain embodiments of the methods of engineering antibodies of the invention, mutations can be introduced randomly or selectively along all or part of an anti-LRP6 antibody coding sequence and the resulting modified anti-LRP6 antibodies can be screened for binding activity and/or other functional properties (e.g., specifically binding to LRP6, interfering with LRP6's ability to bind Wnt pathway members (e.g., DKK1 (dickkopf 1), DKK2, DKK4, SOST1, SOSD1 (USAG1), sFRP (soluble Fzd-related protein) 1-4, Wise, or Wnt ligands), and modulating β-catenin phosphorylation and degradation) as described herein. Mutational methods have been described in the art. For example, PCT Publication WO 02/092780 by Short describes methods for creating and screening antibody mutations using saturation mutagenesis, synthetic ligation assembly, or a combination thereof. Alternatively, WO 03/074679 by Lazar et al. describes methods of using computational screening methods to optimize physiochemical properties of antibodies.

### Non-antibody LRP6 binding molecules

The invention further provides LRP6 binding molecules that exhibit functional properties of antibodies but derive their framework and antigen binding portions from other polypeptides (e.g., polypeptides other than those encoded by antibody genes or generated by the recombination of antibody genes in vivo). The antigen binding domains (e.g., LRP6 binding domains) of these binding molecules are generated through a directed evolution process. See U.S. Pat. No. 7,115,396. Molecules that have an overall fold similar to that of a variable domain of an antibody (an "immunoglobulin-like" fold) are appropriate scaffold proteins. Scaffold proteins suitable for deriving antigen binding molecules include fibronectin or a fibronectin dimer, tenascin, N-cadherin, E-cadherin, ICAM, titin, GCSF-receptor, cytokine receptor, glycosidase inhibitor, antibiotic chromoprotein, myelin membrane adhesion molecule P0, CD8, CD4, CD2, class I MHC, T-cell antigen receptor, CD1, C2 and I-set domains of VCAM-1, I-set immunoglobulin domain of myosin-binding protein C, I-set immunoglobulin domain of myosin-binding protein H, I-set immunoglobulin domain of telokin, NCAM, twitchin, neuroglian, growth hormone receptor, erythropoietin receptor, prolactin receptor, interferon-gamma receptor, β-galactosidase/glucuronidase, β-glucuronidase, transglutaminase, T-cell antigen receptor, superoxide dismutase, tissue factor domain, cytochrome F, green fluorescent protein, GroEL, and thaumatin.

The antigen binding domain (e.g., the immunoglobulin-like fold) of the non-antibody binding molecule can have a molecular mass less than 10 kD or greater than 7.5 kD (e.g., a molecular mass between 7.5-10 kD). The protein used to derive the antigen binding domain is a naturally occurring mammalian protein (e.g., a human protein), and the antigen binding domain includes up to 50% (e.g., up to 34%, 25%, 20%, or 15%), mutated amino acids as compared to the immunoglobulin-like fold of the protein from which it is derived. The domain having the immunoglobulin-like fold generally consists of 50-150 amino acids (e.g., 40-60 amino acids).

To generate non-antibody binding molecules, a library of clones is created in which sequences in regions of the scaffold protein that form antigen binding surfaces (e.g., regions analogous in position and structure to CDRs of an antibody variable domain immunoglobulin fold) are randomized. Library clones are tested for specific binding to the antigen of interest (e.g., hLRP6) and for other functions (e.g., inhibition of biological activity of LRP6). Selected clones can be used as the basis for further randomization and selection to produce derivatives of higher affinity for the antigen. One example of a selection protocol is described in U.S. Pat. No. 6,207,446.

High affinity binding molecules are generated, for example, using the tenth module of fibronectin III (¹⁰Fn3) as the scaffold. A library is constructed for each of three CDR-like loops of ¹⁰FN3 at residues 23-29, 52-55, and 78-87. To construct each library, DNA segments encoding sequence overlapping each CDR-like region are randomized by oligonucleotide synthesis. Techniques for producing selectable ¹⁰Fn3 libraries are described in U.S. Pat. Nos. 6,818,418 and 7,115,396; Roberts and Szostak, 1997 Proc. Natl. Acad. Sci USA 94:12297; U.S. Pat. No. 6,261,804; U.S. Pat. No. 6,258,558; and Szostak et al. WO98/31700.

Non-antibody binding molecules can be produces as dimers or multimers to increase avidity for the target antigen. For example, the antigen binding domain is expressed as a fusion with a constant region (Fc) of an antibody that forms Fc-Fc dimers. See, e.g., U.S. Pat. No. 7,115,396.

### Nucleic acid molecules encoding antibodies of the invention

Another aspect of the invention pertains to nucleic acid molecules that encode the LRP6 binding molecules of the invention. The nucleic acids may be present in whole cells, in a cell lysate, or may be nucleic acids in a partially purified or substantially pure form. A nucleic acid is "isolated" or "rendered substantially pure" when purified away from other cellular components or other contaminants, e.g., other cellular nucleic acids or proteins, by standard techniques, including alkaline/SDS treatment, CsCl banding, column chromatography, agarose gel electrophoresis and others well known in the art. See, F. Ausubel, et al., ed. 1987 Current Protocols in Molecular Biology, Greene Publishing and Wiley Interscience, New York. A nucleic acid of the invention can be, for example, DNA or RNA and may or may not contain intronic sequences. In an embodiment, the nucleic acid is a cDNA molecule. The nucleic acid may be present in a vector such as a phage display vector, or in a recombinant plasmid vector.

Nucleic acids of the invention can be obtained using standard molecular biology techniques. For antibodies expressed by hybridomas (e.g., hybridomas prepared from transgenic mice carrying human immunoglobulin genes as described further below), cDNAs encoding the light and heavy chains of the antibody made by the hybridoma can be obtained by standard PCR amplification or cDNA cloning techniques. For antibodies obtained from an immunoglobulin gene library (e.g., using phage display techniques), nucleic acid encoding the antibody can be recovered from various phage clones that are members of the library.

Once DNA fragments encoding V_{H} and V_{L} segments are obtained, these DNA fragments can be further manipulated by standard recombinant DNA techniques, for example to convert the variable region genes to full-length antibody chain genes, to Fab fragment genes or to an scFv gene. In these manipulations, a V_{L}- or V_{H}-encoding DNA fragment is operatively linked to another DNA molecule, or to a fragment encoding another protein, such as an antibody constant region or a flexible linker. The term "operatively linked", as used in this context, is intended to mean that the two DNA fragments are joined in a functional manner, for example, such that the amino acid sequences encoded by the two DNA fragments remain in-frame, or such that the protein is expressed under control of a desired promoter.

The isolated DNA encoding the V_{H} region can be converted to a full-length heavy chain gene by operatively linking the V_{H}-encoding DNA to another DNA molecule encoding heavy chain constant regions (CH1, CH2 and CH3). The sequences of human heavy chain constant region genes are known in the art (see e.g., Kabat et al., 1991 Sequences of Proteins of Immunological Interest, Fifth Edition, U.S. Department of Health and Human Services, NIH Publication No. 91-3242) and DNA fragments encompassing these regions can be obtained by standard PCR amplification. The heavy chain constant region can be an IgG1, IgG2, IgG3, IgG4, IgA, IgE, IgM or IgD constant region. For a Fab fragment heavy chain gene, the V_{H}-encoding DNA can be operatively linked to another DNA molecule encoding only the heavy chain CH1 constant region.

The isolated DNA encoding the V_{L} region can be converted to a full-length light chain gene (as well as to a Fab light chain gene) by operatively linking the V_{L}-encoding DNA to another DNA molecule encoding the light chain constant region, CL. The sequences of human light chain constant region genes are known in the art (see e.g., Kabat et al., 1991 Sequences of Proteins of Immunological Interest, Fifth Edition, U.S. Department of Health and Human Services, NIH Publication No. 91-3242) and DNA fragments encompassing these regions can be obtained by standard PCR amplification. The light chain constant region can be a kappa or a lambda constant region.

To create an scFv gene, the V_{H}- and V_{L}-encoding DNA fragments are operatively linked to another fragment encoding a flexible linker, e.g., encoding the amino acid sequence (Gly4 -Ser)₃, such that the V_{H} and V_{L} sequences can be expressed as a contiguous single-chain protein, with the V_{L} and V_{H} regions joined by the flexible linker (see e.g., Bird et al., 1988 Science 242:423-426; Huston et al., 1988 Proc. Natl. Acad. Sci. USA 85:5879-5883; McCafferty et al., 1990 Nature 348:552-554).

### Monoclonal Antibody Generation

Monoclonal antibodies (mAbs) can be produced by a variety of techniques, including conventional monoclonal antibody methodology e.g., the standard somatic cell hybridization technique of Kohler and Milstein (1975 Nature, 256:495), or using library display methods, such as phage display.

An animal system for preparing hybridomas is the murine system. Hybridoma production in the mouse is a well established procedure. Immunization protocols and techniques for isolation of immunized splenocytes for fusion are known in the art. Fusion partners (e.g., murine myeloma cells) and fusion procedures are also known.

Chimeric or humanized antibodies of the present invention can be prepared based on the sequence of a murine monoclonal antibody prepared as described above. DNA encoding the heavy and light chain immunoglobulins can be obtained from the murine hybridoma of interest and engineered to contain non-murine (e.g., human) immunoglobulin sequences using standard molecular biology techniques. For example, to create a chimeric antibody, the murine variable regions can be linked to human constant regions using methods known in the art (see e.g., U.S. Pat. No. 4,816,567 to Cabilly et al.). To create a humanized antibody, the murine CDR regions can be inserted into a human framework using methods known in the art. See e.g., U.S. Pat. No. 5,225,539, and U.S. Pat. Nos. 5,530,101; 5,585,089; 5,693,762 and 6,180,370.

In a certain embodiment, the antibodies of the invention are human monoclonal antibodies. Such human monoclonal antibodies directed against LRP6 can be generated using transgenic or transchromosomic mice carrying parts of the human immune system rather than the mouse system. These transgenic and transchromosomic mice include mice referred to herein as HuMAb mice and KM mice, respectively, and are collectively referred to herein as "human Ig mice."

The HuMAb mouse® (Medarex, Inc.) contains human immunoglobulin gene miniloci that encode un-rearranged human heavy (µ and γ) and κ light chain immunoglobulin sequences, together with targeted mutations that inactivate the endogenous µ and κ chain loci (see, e.g., Lonberg et al., 1994 Nature 368(6474): 856-859). Accordingly, the mice exhibit reduced expression of mouse IgM or κ, and in response to immunization, the introduced human heavy and light chain transgenes undergo class switching and somatic mutation to generate high affinity human IgGκ monoclonal (Lonberg, N. et al., 1994 supra; reviewed in Lonberg, N., 1994 Handbook of Experimental Pharmacology 113:49-101; Lonberg, N. and Huszar, D., 1995 Intern. Rev. Immunol.13: 65-93, and Harding, F. and Lonberg, N., 1995 Ann. N. Y. Acad. Sci. 764:536-546). The preparation and use of HuMAb mice, and the genomic modifications carried by such mice, is further described in Taylor, L. et al., 1992 Nucleic Acids Research 20:6287-6295; Chen, J. et at., 1993 International Immunology 5: 647-656; Tuaillon et al., 1993 Proc. Natl. Acad. Sci. USA 94:3720-3724; Choi et al., 1993 Nature Genetics 4:117-123; Chen, J. et al., 1993 EMBO J. 12: 821-830; Tuaillon et al., 1994 J. Immunol. 152:2912-2920; Taylor, L. et al., 1994 International Immunology 579-591; and Fishwild, D. et al., 1996 Nature Biotechnology 14: 845-851, the contents of all of which are hereby specifically incorporated by reference in their entirety. See further, U.S. Pat. Nos. 5,545,806; 5,569,825; 5,625,126; 5,633,425; 5,789,650; 5,877,397; 5,661,016; 5,814,318; 5,874,299; and 5,770,429; all to Lonberg and Kay; U.S. Pat. No. 5,545,807 to Surani et al.; PCT Pub. Nos. WO 92103918, WO 93/12227, WO 94/25585, WO 97113852, WO 98/24884 and WO 99/45962, all to Lonberg and Kay; and PCT Pub. No. WO 01/14424 to Korman et al.

In another embodiment, human antibodies of the invention can be raised using a mouse that carries human immunoglobulin sequences on transgenes and transchomosomes, such as a mouse that carries a human heavy chain transgene and a human light chain transchromosome. Such mice, referred to herein as "KM mice", are described in detail in WO 02/43478.

Still further, alternative transgenic animal systems expressing human immunoglobulin genes are available in the art and can be used to raise anti-LRP6 antibodies of the invention. For example, an alternative transgenic system referred to as the Xenomouse® (Abgenix, Inc.) can be used. Such mice are described in, e.g., U.S. Pat. Nos. 5,939,598; 6,075,181; 6,114,598; 6, 150,584 and 6,162,963 to Kucherlapati et al.

Moreover, alternative transchromosomic animal systems expressing human immunoglobulin genes are available in the art and can be used to raise anti-LRP6 antibodies of the invention. For example, mice carrying both a human heavy chain transchromosome and a human light chain tranchromosome, referred to as "TC mice" can be used; such mice are described in Tomizuka et al., 2000 Proc. Natl. Acad. Sci. USA 97:722-727. Furthermore, cows carrying human heavy and light chain transchromosomes have been described in the art (Kuroiwa et al., 2002 Nature Biotechnology 20:889-894) and can be used to raise anti-LRP6 antibodies of the invention.

Human monoclonal antibodies of the invention can also be prepared using phage display methods for screening libraries of human immunoglobulin genes. Such phage display methods for isolating human antibodies are established in the art. See for example: U.S. Pat. Nos. 5,223,409; 5,403,484; and 5,571,698 to Ladner et al.; U.S. Pat. Nos. 5,427,908 and 5,580,717 to Dower et al.; U.S. Pat. Nos. 5,969,108 and 6,172,197 to McCafferty et al.; and U.S. Pat. Nos. 5,885,793; 6,521,404; 6,544,731; 6,555,313; 6,582,915 and 6,593,081 to Griffiths et al. Libraries can be screened for binding to full length LRP6 or to a particular epitope of LRP6.

Human monoclonal antibodies of the invention can also be prepared using SCID mice into which human immune cells have been reconstituted such that a human antibody response can be generated upon immunization. Such mice are described in, for example, U.S. Pat. Nos. 5,476,996 and 5,698,767 to Wilson et al.

### Generation of human monoclonal antibodies in Human Ig Mice

Purified recombinant human LRP6 expressed in prokaryotic cells (e.g., *E. coli*) or eukaryotic cells (e.g., mammalian cells, e.g., HEK293 cells) can be used as the antigen. The protein can be conjugated to a carrier, such as keyhole limpet hemocyanin (KLH).

Fully human monoclonal antibodies to LRP6 are prepared using HCo7, HCo12 and HCo17 strains of HuMab transgenic mice and the KM strain of transgenic transchromosomic mice, each of which express human antibody genes. In each of these mouse strains, the endogenous mouse kappa light chain gene can be homozygously disrupted as described in Chen et al., 1993 EMBO J.12:811-820 and the endogenous mouse heavy chain gene can be homozygously disrupted as described in Example 1 of WO 01109187. Each of these mouse strains carries a human kappa light chain transgene, KCo5, as described in Fishwild et al., 1996 Nature Biotechnology 14:845-851. The HCo7 strain carries the HCo7 human heavy chain transgene as described in U.S. Pat. Nos. 5,545,806; 5,625,825; and 5,545,807. The HCo12 strain carries the HCo12 human heavy chain transgene as described in Example 2 of WO 01/09187. The HCo17 stain carries the HCo17 human heavy chain transgene. The KNM strain contains the SC20 transchromosome as described in WO 02/43478.

To generate fully human monoclonal antibodies to LRP6, HuMab mice and KM mice are immunized with purified recombinant LRP6, an LRP6 fragment, or a conjugate thereof (e.g., LRP6-KLH) as antigen. General immunization schemes for HuMab mice are described in Lonberg, N. et al., 1994 Nature 368(6474): 856-859; Fishwild, D. et al., 1996 Nature Biotechnology 14:845-851 and WO 98/24884. The mice are 6-16 weeks of age upon the first infusion of antigen. A purified recombinant preparation (5-50 µg) of the antigen is used to immunize the HuMab mice and KM mice in the peritoneal cavity, subcutaneously (Sc) or by footpad injection.

Transgenic mice are immunized twice with antigen in complete Freund's adjuvant or Ribi adjuvant either in the peritoneal cavity (IP), subcutaneously (Sc) or by footpad (FP), followed by 3-21 days IP, Sc or FP immunization (up to a total of 11 immunizations) with the antigen in incomplete Freund's or Ribi adjuvant. The immune response is monitored by retroorbital bleeds. The plasma is screened by ELISA, and mice with sufficient titers of anti-LRP6 human immunogolobulin are used for fusions. Mice are boosted intravenously with antigen 3 and 2 days before sacrifice and removal of the spleen. Typically, 10-35 fusions for each antigen are performed. Several dozen mice are immunized for each antigen. A total of 82 mice of the HCo7, HCo12, HCo17 and KM mice strains are immunized with LRP6.

To select HuMab or KM mice producing antibodies that bound LRP6, sera from immunized mice can be tested by ELISA as described by Fishwild, D. et al., 1996. Briefly, microtiter plates are coated with purified recombinant LRP6 at 1-2 µg /ml in PBS, 50 µl/wells incubated 4 °C overnight then blocked with 200 µl/well of 5% chicken serum in PBS/Tween (0.05%). Dilutions of plasma from LRP6-immunized mice are added to each well and incubated for 1-2 hours at ambient temperature. The plates are washed with PBS/Tween and then incubated with a goat-anti-human IgG Fc polyclonal antibody conjugated with horseradish peroxidase (HRP) for 1 hour at room temperature. After washing, the plates are developed with ABTS substrate (Sigma, A-1888, 0.22 mg/ml) and analyzed by spectrophotometer at OD 415-495. Splenocytes of mice that developed the highest titers of anti-LRP6 antibodies are used for fusions. Fusions are performed and hybridoma supernatants are tested for anti-LRP6 activity by ELISA.

The mouse splenocytes, isolated from the HuMab mice and KM mice, are fused with PEG to a mouse myeloma cell line based upon standard protocols. The resulting hybridomas are then screened for the production of antigen-specific antibodies. Single cell suspensions of splenic lymphocytes from immunized mice are fused to one-fourth the number of SP2/0 nonsecreting mouse myeloma cells (ATCC, CRL 1581) with 50% PEG (Sigma). Cells are plated at approximately 1x10⁵/well in flat bottom microtiter plates, followed by about two weeks of incubation in selective medium containing 10% fetal bovine serum, 10% P388D 1(ATCC, CRL TIB-63) conditioned medium, 3-5% Origen® (IGEN) in DMEM (Mediatech, CRL 10013, with high glucose, L-glutamine and sodium pyruvate) plus 5 mM HEPES, 0.055 mM 2-mercaptoethanol, 50 µg/ml gentamycin and 1x HAT (Sigma, CRL P-7185). After 1-2 weeks, cells are cultured in medium in which the HAT is replaced with HT. Individual wells are then screened by ELISA for human anti-LRP6 monoclonal IgG antibodies. Once extensive hybridoma growth occurred, medium is monitored usually after 10-14 days. The antibody secreting hybridomas are replated, screened again and, if still positive for human IgG, anti-LRP6 monoclonal antibodies are subcloned at least twice by limiting dilution. The stable subclones are then cultured in vitro to generate small amounts of antibody in tissue culture medium for further characterization.

### Generation of hybridomas producing human monoclonal antibodies

To generate hybridomas producing human monoclonal antibodies of the invention, splenocytes and/or lymph node cells from immunized mice can be isolated and fused to an appropriate immortalized cell line, such as a mouse myeloma cell line. The resulting hybridomas can be screened for the production of antigen-specific antibodies. For example, single cell suspensions of splenic lymphocytes from immunized mice can be fused to one-sixth the number of P3X63-Ag8.653 nonsecreting mouse myeloma cells (ATCC, CRL 1580) with 50% PEG. Cells are plated at approximately 2 x 145 in flat bottom microtiter plates, followed by a two week incubation in selective medium containing 20% fetal Clone Serum, 18% "653" conditioned media, 5% Origen® (IGEN), 4 mM L-glutamine, 1 mM sodium pyruvate, 5mM HEPES, 0:055 mM 2-mercaptoethanol, 50 units/ml penicillin, 50 µg/ml streptomycin, 50 µg/ml gentamycin and 1X HAT (Sigma; the HAT is added 24 hours after the fusion). After approximately two weeks, cells can be cultured in medium in which the HAT is replaced with HT. Individual wells can then be screened by ELISA for human monoclonal IgM and IgG antibodies. Once extensive hybridoma growth occurs, medium can be observed usually after 10-14 days. The antibody secreting hybridomas can be replated, screened again, and if still positive for human IgG, the monoclonal antibodies can be subcloned at least twice by limiting dilution. The stable subclones can then be cultured in vitro to generate small amounts of antibody in tissue culture medium for characterization.

To purify human monoclonal antibodies, selected hybridomas can be grown in two-liter spinner-flasks for monoclonal antibody purification. Supernatants can be filtered and concentrated before affinity chromatography with protein A-sepharose (Pharmacia, Piscataway, N.J.). Eluted IgG can be checked by gel electrophoresis and high performance liquid chromatography to ensure purity. The buffer solution can be exchanged into PBS, and the concentration can be determined by OD₂₈₀ using an extinction coefficient of 1.43. The monoclonal antibodies can be aliquoted and stored at -80° C.

### Generation of transfectomas producing monoclonal antibodies

Antibodies of the invention also can be produced in a host cell transfectoma using, for example, a combination of recombinant DNA techniques and gene transfection methods as is well known in the art (e.g., Morrison, 1985 Science 229:1202).

For example, to express the antibodies, or antibody fragments thereof, DNAs encoding partial or full-length light and heavy chains, can be obtained by standard molecular biology techniques (e.g., PCR amplification or cDNA cloning using a hybridoma that expresses the antibody of interest) and the DNAs can be inserted into expression vectors such that the genes are operatively linked to transcriptional and translational control sequences. In this context, the term "operatively linked" is intended to mean that an antibody gene is ligated into a vector such that transcriptional and translational control sequences within the vector serve their intended function of regulating the transcription and translation of the antibody gene. The expression vector and expression control sequences are chosen to be compatible with the expression host cell used. The antibody light chain gene and the antibody heavy chain gene can be inserted into separate vector or, more typically, both genes are inserted into the same expression vector. The antibody genes are inserted into the expression vector by standard methods (e.g., ligation of complementary restriction sites on the antibody gene fragment and vector, or blunt end ligation if no restriction sites are present). The light and heavy chain variable regions of the antibodies described herein can be used to create full-length antibody genes of any antibody isotype by inserting them into expression vectors already encoding heavy chain constant and light chain constant regions of the desired isotype such that the V_{H} segment is operatively linked to the CH segment(s) within the vector and the V_{L} segment is operatively linked to the CL segment within the vector. Additionally or alternatively, the recombinant expression vector can encode a signal peptide that facilitates secretion of the antibody chain from a host cell. The antibody chain gene can be cloned into the vector such that the signal peptide is linked in frame to the amino terminus of the antibody chain gene. The signal peptide can be an immunoglobulin signal peptide or a heterologous signal peptide (i.e., a signal peptide from a non-immunoglobulin protein).

In addition to the antibody chain genes, the recombinant expression vectors of the invention carry regulatory sequences that control the expression of the antibody chain genes in a host cell. The term "regulatory sequence" is intended to include promoters, enhancers and other expression control elements (e.g., polyadenylation signals) that control the transcription or translation of the antibody chain genes. Such regulatory sequences are described, for example, in Goeddel (Gene Expression Technology. 1990 Methods in Enzymology 185, Academic Press, San Diego, CA). It will be appreciated by those skilled in the art that the design of the expression vector, including the selection of regulatory sequences, may depend on such factors as the choice of the host cell to be transformed, the level of expression of protein desired, etc. Regulatory sequences for mammalian host cell expression include viral elements that direct high levels of protein expression in mammalian cells, such as promoters and/or enhancers derived from cytomegalovirus (CMV), Simian Virus 40 (SV40), adenovirus (e.g., the adenovirus major late promoter (AdMLP)), and polyoma. Alternatively, nonviral regulatory sequences may be used, such as the ubiquitin promoter or P-globin promoter. Still further, regulatory elements composed of sequences from different sources, such as the SRa promoter system, which contains sequences from the SV40 early promoter and the long terminal repeat of human T cell leukemia virus type 1 (Takebe et al., 1988 Mol. Cell. Biol. 8:466-472).

In addition to the antibody chain genes and regulatory sequences, the recombinant expression vectors of the invention may carry additional sequences, such as sequences that regulate replication of the vector in host cells (e.g., origins of replication) and selectable marker genes. The selectable marker gene facilitates selection of host cells into which the vector has been introduced (see, e.g., U.S. Pat. Nos. 4,399,216; 4,634,665; and 5,179,017, all by Axel et al.). For example, typically the selectable marker gene confers resistance to drugs, such as G418, hygromycin or methotrexate, on a host cell into which the vector has been introduced. Selectable marker genes include the dihydrofolate reductase (DHFR) gene (for use in dhfr- host cells with methotrexate selection/amplification) and the neo gene (for G418 selection).

For expression of the light and heavy chains, the expression vector(s) encoding the heavy and light chains is transfected into a host cell by standard techniques. The various forms of the term "transfection" are intended to encompass a wide variety of techniques commonly used for the introduction of exogenous DNA into a prokaryotic or eukaryotic host cell, e.g., electroporation, calcium-phosphate precipitation, DEAE-dextran transfection and the like. It is theoretically possible to express the antibodies of the invention in either prokaryotic or eukaryotic host cells. Expression of antibodies in eukaryotic cells, in particular mammalian host cells, is discussed because such eukaryotic cells, and in particular mammalian cells, are more likely than prokaryotic cells to assemble and secrete a properly folded and immunologically active antibody. Prokaryotic expression of antibody genes has been reported to be ineffective for production of high yields of active antibody (Boss and Wood, 1985 Immunology Today 6:12-13).

Mammalian host cells for expressing the recombinant antibodies of the invention include Chinese Hamster Ovary (CHO cells) (including dhfr- CHO cells, described Urlaub and Chasin, 1980 Proc. Natl. Acad. Sci. USA 77:4216-4220 used with a DH FR selectable marker, e.g., as described in Kaufman and Sharp, 1982 Mol. Biol. 159:601-621, NS0 myeloma cells, COS cells and SP2 cells. In particular, for use with NS0 myeloma cells, another expression system is the GS gene expression system shown in WO 87/04462, WO 89/01036 and EP 338,841. When recombinant expression vectors encoding antibody genes are introduced into mammalian host cells, the antibodies are produced by culturing the host cells for a period of time sufficient to allow for expression of the antibody in the host cells or secretion of the antibody into the culture medium in which the host cells are grown. Antibodies can be recovered from the culture medium using standard protein purification methods.

### Bispecific molecules

In another aspect, the present invention features bispecific molecules comprising an LRP6 binding molecule (e.g., an anti-LRP6 antibody, or a fragment thereof) of the invention. A LRP6 binding molecule of the invention can be derivatized or linked to another functional molecule, e.g., another peptide or protein (e.g., another antibody or ligand for a receptor) to generate a bispecific molecule that binds to at least two different binding sites or target molecules. The LRP6 binding molecule of the invention may in fact be derivatized or linked to more than one other functional molecule to generate multi-specific molecules that bind to more than two different binding sites and/or target molecules; such multi-specific molecules are also intended to be encompassed by the term "bispecific molecule" as used herein. To create a bispecific molecule of the invention, an antibody of the invention can be functionally linked (e.g., by chemical coupling, genetic fusion, noncovalent association or otherwise) to one or more other binding molecules, such as another antibody, antibody fragment, peptide or binding mimetic, such that a bispecific molecule results.

Accordingly, the present invention includes bispecific molecules comprising at least one first binding specificity for one LRP6 epitope and a second binding specificity for a second target epitope, on LRP6 or on another protein target. By way of non-limiting example, a bispecific molecule of the invention is capable of binding to both propeller 1 and propeller 3 of LRP6.

In one embodiment, the bispecific molecules of the invention comprise as a binding specificity at least one antibody, or an antibody fragment thereof, including, e.g., an Fab, Fab', F(ab')₂, Fv, or a single chain Fv. The antibody may also be a light chain or heavy chain dimer, or any minimal fragment thereof such as a Fv or a single chain construct as described in Ladner et al. U.S. Pat. No. 4,946,778, the contents of which is expressly incorporated by reference.

The bispecific molecules of the present invention can be prepared by conjugating the constituent binding specificities using methods known in the art. For example, each binding specificity of the bispecific molecule can be generated separately and then conjugated to one another. When the binding specificities are proteins or peptides, a variety of coupling or cross-linking agents can be used for covalent conjugation. Examples of cross-linking agents include protein A, carbodiimide, N-succinimidyl-S-acetyl-thioacetate (SATA), 5,5'-dithiobis(2-nitrobenzoic acid) (DTNB), o-phenylenedimaleimide (oPDM), N-succinimidyl-3-(2-pyridyldithio)propionate (SPDP), and sulfosuccinimidyl 4-(N-maleimidomethyl) cyclohaxane-1-carboxylate (sulfo-SMCC) (see e.g., Karpovsky et al., 1984 J. Exp. Med. 160:1686; Liu et al., 1985 Proc. Natl. Acad. Sci. USA 82:8648). Other methods include those described in Paulus, 1985 Behring Ins. Mitt. No. 78,118-132; Brennan et al., 1985 Science 229:81-83), and Glennie et al., 1987 J. Immunol. 139: 2367-2375). Conjugating agents are SATA and sulfo-SMCC, both available from Pierce Chemical Co. (Rockford, IL).

When the binding specificities are antibodies, they can be conjugated by sulfhydryl bonding of the C-terminus hinge regions of the two heavy chains. In a particular embodiment, the hinge region is modified to contain an odd number of sulfhydryl residues, for example one, prior to conjugation.

Alternatively, both binding specificities can be encoded in the same vector and expressed and assembled in the same host cell. This method is particularly useful where the bispecific molecule is a mAb x mAb, mAb x Fab, Fab x F(ab')₂ or ligand x Fab fusion protein. A bispecific molecule of the invention can be a single chain molecule comprising one single chain antibody and a binding determinant, or a single chain bispecific molecule comprising two binding determinants. Bispecific molecules may comprise at least two single chain molecules. Methods for preparing bispecific molecules are described for example in U.S. Pat. Nos. 5,260,203; 5,455,030; 4,881,175; 5,132,405; 5,091,513; 5,476,786; 5,013,653; 5,258,498; and 5,482,858.

Binding of the bispecific molecules to their specific targets can be confirmed by, for example, enzyme-linked immunosorbent assay (ELISA), radioimmunoassay (REA), FACS analysis, bioassay (e.g., growth inhibition), or Western Blot assay. Each of these assays generally detects the presence of protein-antibody complexes of particular interest by employing a labeled reagent (e.g., an antibody) specific for the complex of interest.

### Functional assays

The functional characteristics of LRP6 binding molecules can be tested in vitro and in vivo. For example, binding molecules can be tested for the ability to interfere with LRP6's ability to bind Wnt pathway members (e.g., DKK1 (dickkopf 1), DKK2, DKK4, SOST1, SOSD1 (USAG1), sFRP (soluble Fzd-related protein) 1-4, Wise, or Wnt ligands), or to modulate such biological processes as β-catenin phosphorylation and degradation, tumor cell growth, apoptosis, regulation of bone mineral density, and insulin secretion.

LRP6 binding to Wnt pathway members (e.g., DKK1 (dickkopf 1), DKK2, DKK4, SOST1, SOSD1 (USAG1), sFRP (soluble Fzd-related protein) 1-4, Wise, or Wnt ligands) can be detected using Biacore® by immobilizing said Wnt pathway members to a solid support and detecting soluble LRP6 binding thereto. Alternatively, LRP6 can be immobilized, and Wnt pathway member binding thereto can be detected. LRP6/Wnt pathway members binding can also be analyzed by ELISA (e.g., by detecting LRP6 binding to immobilized Wnt pathway members), or by fluorescence resonance energy transfer (FRET). To perform FRET, fluorophore-labeled LRP6 binding to Wnt pathway members in solution can be detected (see, for example, U.S. Pat. No. 5,631,169).

LRP6 binding to Wnt pathway members (e.g., DKK1 (dickkopf 1), DKK2, DKK4, SOST1, SOSD1 (USAG1), sFRP (soluble Fzd-related protein) 1-4, Wise, or Wnt ligands) can also be detected via "liquid binding" methods, i.e., measuring affinity in liquid setting, instead of in an immobilized environment. Said methods are offered by, e.g., BioVeris (now Roche).

LRP6 binding to said Wnt pathway members can be detected by coimmunoprecipitation (Lagace et al., 2006 J. Clin. Inv. 116(11):2995-3005). To examine LRP6-Wnt pathway members binding in this manner, HepG2 cells are cultured in sterol-depleted medium for 18 hours. Purified LRP6 is added to the medium in the presence of 0.1 mM chloroquine and the cells are incubated for one hour. Cells are lysed in mild detergent (1% digitonin w/vol). LRP6 or a Wnt pathway member is immunoprecipitated from cell lysates, separated by SDS-PAGE, and immunoblotted to detect the presence of coimmunoprecipitated said Wnt pathway member or LRP6, respectively (Lagace et al., 2006 J. Clin. Inv. 116(11):2995-3005). These assays may be conducted with a mutant form of LRP6 that binds to Wnt pathway member with a higher avidity (Lagace et al., 2006, supra).

LRP6 binding molecules can be tested for the ability to increase or decrease Wnt pathway members levels within said cells. For example, cells are cultured in sterol-depleted medium (DMEM supplemented with 100 U/ml penicillin, 100 µg/ml streptomycin sulfate, and 1 g/l glucose, 5% (vol/vol) newborn calf lipoprotein-deficient serum (NCLPDS), 10 µM sodium compactin, and 50 µM sodium mevalonate) for 18 hours to induce Wnt pathway member expression. Purified LRP6 (5 µg/ml) is added to the medium. Wnt pathway member levels in cells harvested at 0, 0.5, 1, 2, and 4 hours after addition of LRP6 is determined (Lagace et al., 2006 J. Clin. Inv. 116(11):2995-3005). Wnt pathway members levels can be determined by flow cytometry, FRET, immunoblotting, or other means.

LRP6 binding molecules can be tested for the ability to increase or decrease Wnt induced LRP6 phosphorylation, and β-catenin stabilization, reporter gene or target gene expression.

LRP6 binding to Wnt pathway members can be detected by production of cell membrane preparations containing LRP6 and tested for ability to bind said preparations compared to cell membrane preparations not containing LRP6. Or by FACS analysis of cells with endogenous LRP6 or cells with overexpressed LRP6.

Anti-LRP6 Abs may be tested by their ability to inhibit growth of Wnt-dependent tumors in mice. For example, inhibition of tumor growth of WNT1-MMTV tumors by anti-LRP6 Abs. Inhibition of tumor growth within tumor xenograft models (e.g., SCID xenograft models, orthotopic xenograft models, etc.) is another environment in which anti-LRP6 Abs may be tested by their ability to inhibit growth of Wnt-dependent tumors.

Anti-LRP6 Abs may be tested by their ability to inhibit growth of Wnt dependent tumors in vitro, such as inhibition of colony formation in soft agar.

### Pharmaceutical compositions

In another aspect, the present invention provides a composition, e.g., a pharmaceutical composition, containing one or a combination of LRP6 binding molecules (e.g., monoclonal antibodies, or antigen-binding portion(s) thereof), of the present invention, formulated together with a pharmaceutically acceptable carrier. Such compositions may include one or a combination of (e.g., two or more different) binding molecules. For example, a pharmaceutical composition of the invention can comprise a combination of antibodies or agents that bind to different epitopes on the target antigen or that have complementary activities.

Pharmaceutical compositions of the invention also can be administered in combination therapy, i.e., combined with other agents. For example, for cancer therapy, the combination therapy can include an anti-LRP6 antibody (e.g., an antagonizing LRP6 antibody) combined with at least one other chemotherapeutic agent. Likewise, for diabetic therapy, the combination therapy can include an anti-LRP6 antibody (e.g., an agonizing LRP6 antibody) combined with at least one other insulin secretion enhancer, such as nateglinides or repaglinides. Likewise, for osteoporotic therapy, the combination therapy can include an anti-LRP6 antibody (e.g., an agonizing LRP6 antibody) combined with at least one other agent capable of increasing bone density, such as calcium, vitamin D, or bisphosphonates. Examples of therapeutic agents that can be used in combination therapy are described in greater detail below in the section on uses of the agents of the invention.

As used herein, "pharmaceutically acceptable carrier" includes any and all solvents, dispersion media, coatings, antibacterial and antifungal agents, isotonic and absorption delaying agents, and the like that are physiologically compatible. The carrier should be suitable for intravenous, intramuscular, subcutaneous, parenteral, spinal or epidermal administration (e.g., by injection or infusion). Depending on the route of administration, the active compound may be coated in a material to protect the compound from the action of acids and other natural conditions that may inactivate the compound.

The pharmaceutical compounds of the invention may include one or more pharmaceutically acceptable salts. A "pharmaceutically acceptable salt" refers to a salt that retains the desired biological activity of the parent compound and does not impart any undesired toxicological effects (see e.g., Berge, S.M., et al., 1977 J. Pharm. Sci. 66:1-19). Examples of such salts include acid addition salts and base addition salts. Acid addition salts include those derived from nontoxic inorganic acids, such as hydrochloric, nitric, phosphoric, sulfuric, hydrobromic, hydroiodic, phosphorous and the like, as well as from nontoxic organic acids such as aliphatic mono- and di-carboxylic acids, phenyl-substituted alkanoic acids, hydroxy alkanoic acids, aromatic acids, aliphatic and aromatic sulfonic acids and the like. Base addition salts include those derived from alkaline earth metals, such as sodium, potassium, magnesium, calcium and the like, as well as from nontoxic organic amines, such as N,N'-dibenzylethylenediamine, N-methylglucamine, chloroprocaine, choline, diethanolamine, ethylenediamine, procaine and the like.

A pharmaceutical composition of the invention also may include a pharmaceutically acceptable anti-oxidant. Examples of pharmaceutically acceptable antioxidants include: water soluble antioxidants, such as ascorbic acid, cysteine hydrochloride, sodium bisulfate, sodium metabisulfite, sodium sulfite and the like; oil-soluble antioxidants, such as ascorbyl palmitate, butylated hydroxyanisole (BHA), butylated hydroxytoluene (BHT), lecithin, propyl gallate, alpha-tocopherol, and the like; and metal chelating agents, such as citric acid, ethylenediamine tetraacetic acid (EDTA), sorbitol, tartaric acid, phosphoric acid, and the like.

Examples of suitable aqueous and nonaqueous carriers that may be employed in the pharmaceutical compositions of the invention include water, ethanol, polyols (such as glycerol, propylene glycol, polyethylene glycol, and the like), and suitable mixtures thereof, vegetable oils, such as olive oil, and injectable organic esters, such as ethyl oleate. Proper fluidity can be maintained, for example, by the use of coating materials, such as lecithin, by the maintenance of the required particle size in the case of dispersions, and by the use of surfactants.

These compositions may also contain adjuvants such as preservatives, wetting agents, emulsifying agents and dispersing agents. Prevention of presence of microorganisms may be ensured both by sterilization procedures, supra, and by the inclusion of various antibacterial and antifungal agents, for example, paraben, chlorobutanol, phenol sorbic acid, and the like. It may also be desirable to include isotonic agents, such as sugars, sodium chloride, and the like into the compositions. In addition, prolonged absorption of the injectable pharmaceutical form may be brought about by the inclusion of agents which delay absorption such as, aluminum monostearate and gelatin.

Pharmaceutically acceptable carriers include sterile aqueous solutions or dispersions and sterile powders for the extemporaneous preparation of sterile injectable solutions or dispersion. The use of such media and agents for pharmaceutically active substances is known in the art. Except insofar as any conventional media or agent is incompatible with the active compound, use thereof in the pharmaceutical compositions of the invention is contemplated. Supplementary active compounds can also be incorporated into the compositions.

Therapeutic compositions typically must be sterile and stable under the conditions of manufacture and storage. The composition can be formulated as a solution, microemulsion, liposome, or other ordered structure suitable to high drug concentration. The carrier can be a solvent or dispersion medium containing, for example, water, ethanol, polyol (for example, glycerol, propylene glycol, and liquid polyethylene glycol, and the like), and suitable mixtures thereof. The proper fluidity can be maintained, for example, by the use of a coating such as lecithin, by the maintenance of the required particle size in the case of dispersion and by the use of surfactants. In many cases, one can include isotonic agents, for example, sugars, polyalcohols such as mannitol, sorbitol, or sodium chloride in the composition. Prolonged absorption of the injectable compositions can be brought about by including in the composition an agent that delays absorption for example, monostearate salts and gelatin.

Sterile injectable solutions can be prepared by incorporating the active compound in the required amount in an appropriate solvent with one or a combination of ingredients enumerated above, as required, followed by sterilization micro filtration. Generally, dispersions are prepared by incorporating the active compound into a sterile vehicle that contains a basic dispersion medium and the required other ingredients from those enumerated above. In the case of sterile powders for the preparation of sterile injectable solutions, the methods of preparation are vacuum drying and freeze-drying (lyophilization) that yield a powder of the active ingredient plus any additional desired ingredient from a previously sterile-filtered solution thereof.

The amount of active ingredient which can be combined with a carrier material to produce a single dosage form will vary depending upon the subject being treated, and the particular mode of administration. The amount of active ingredient which can be combined with a carrier material to produce a single dosage form will generally be that amount of the composition which produces a therapeutic effect. Generally, out of one hundred percent, this amount will range from about 0.01 per cent to about ninety-nine percent of active ingredient, from about 0.1 per cent to about 70 per cent, or from about 1 percent to about 30 percent of active ingredient in combination with a pharmaceutically acceptable carrier.

Dosage regimens are adjusted to provide the optimum desired response (e.g., a therapeutic response). For example, a single bolus may be administered, several divided doses may be administered over time or the dose may be proportionally reduced or increased as indicated by the exigencies of the therapeutic situation. It is especially advantageous to formulate parenteral compositions in dosage unit form for ease of administration and uniformity of dosage. Dosage unit form as used herein refers to physically discrete units suited as unitary dosages for the subjects to be treated; each unit contains a predetermined quantity of active compound calculated to produce the desired therapeutic effect in association with the required pharmaceutical carrier. The specification for the dosage unit forms of the invention are dictated by and directly dependent on the unique characteristics of the active compound and the particular therapeutic effect to be achieved, and the limitations inherent in the art of compounding such an active compound for the treatment of sensitivity in individuals.

For administration of the antibody, the dosage ranges from about 0.0001 to 100 mg/kg, and more usually 0.01 to 5 mg/kg, of the host body weight. For example dosages can be 0.3 mg/kg body weight, 1 mg/kg body weight, 3 mg/kg body weight, 5 mg/kg body weight or 10 mg/kg body weight or within the range of 1-10 mg/kg. An exemplary treatment regime entails administration once per week, once every two weeks, once every three weeks, once every four weeks, once a month, once every 3 months or once every three to 6 months. Dosage regimens for LRP6 binding molecule of the invention include 1 mg/kg body weight or 3 mg/kg body weight by intravenous administration, with the antibody being given using one of the following dosing schedules: every four weeks for six dosages, then every three months; every three weeks; 3 mg/kg body weight once followed by 1 mg/kg body weight every three weeks.

In some methods, two or more binding molecules (e.g., monoclonal antibodies) with different binding specificities are administered simultaneously, in which case the dosage of each antibody administered falls within the ranges indicated. The LRP6 binding molecule is usually administered on multiple occasions. Intervals between single dosages can be, for example, weekly, monthly, every three months or yearly. Intervals can also be irregular as indicated by measuring blood levels of binding molecule to LRP6 in the patient. In some methods, dosage is adjusted to achieve a plasma concentration of the LRP6 binding molecule of about 1-1000 µg/ml and in some methods about 25-300 µg/ml.

Alternatively, an LRP6 binding molecule can be administered as a sustained release formulation, in which case less frequent administration is required. Dosage and frequency vary depending on the half-life of the LRP6 binding molecule in the patient. In general, human antibodies show the longest half-life, followed by humanized antibodies, chimeric antibodies, and nonhuman antibodies. The dosage and frequency of administration can vary depending on whether the treatment is prophylactic or therapeutic. In prophylactic applications, a relatively low dosage is administered at relatively infrequent intervals over a long period of time. Some patients continue to receive treatment for the rest of their lives. In therapeutic applications, a relatively high dosage at relatively short intervals is sometimes required until progression of the disease is reduced or terminated or until the patient shows partial or complete amelioration of symptoms of disease. Thereafter, the patient can be administered a prophylactic regime.

Actual dosage levels of the active ingredients in the pharmaceutical compositions of the present invention may be varied so as to obtain an amount of the active ingredient which is effective to achieve the desired therapeutic response for a particular patient, composition, and mode of administration, without being toxic to the patient. The selected dosage level will depend upon a variety of pharmacokinetic factors including the activity of the particular compositions of the present invention employed, or the ester, salt or amide thereof, the route of administration, the time of administration, the rate of excretion of the particular compound being employed, the duration of the treatment, other drugs, compounds and/or materials used in combination with the particular compositions employed, the age, sex, weight, condition, general health and prior medical history of the patient being treated, and like factors well known in the medical arts.

A composition of the present invention can be administered by one or more routes of administration using one or more of a variety of methods known in the art. As will be appreciated by the skilled artisan, the route and/or mode of administration will vary depending upon the desired results. Routes of administration for LRP6 binding molecules of the invention include intravenous, intramuscular, intradermal, intraperitoneal, subcutaneous, spinal or other parenteral routes of administration, for example by injection or infusion. The phrase "parenteral administration" as used herein means modes of administration other than enteral and topical administration, usually by injection, and includes, without limitation, intravenous, intramuscular, intraarterial, intrathecal, intracapsular, intraorbital, intracardiac, intradermal, intraperitoneal, transtracheal, subcutaneous, subcuticular, intraarticular, subcapsular, subarachnoid, intraspinal, epidural and intrastemal injection and infusion.

Alternatively, an LRP6 binding molecule of the invention can be administered by a nonparenteral route, such as a topical, epidermal or mucosal route of administration, for example, intranasally, orally, vaginally, rectally, sublingually or topically.

The active compounds can be prepared with carriers that will protect the compound against rapid release, such as a controlled release formulation, including implants, transdermal patches, and microencapsulated delivery systems. Biodegradable, biocompatible polymers can be used, such as ethylene vinyl acetate, polyanhydrides, polyglycolic acid, collagen, polyorthoesters, and polylactic acid. Many methods for the preparation of such formulations are patented or generally known to those skilled in the art. See, e.g., Sustained and Controlled Release Drug Delivery Systems, J.R. Robinson, ed., Marcel Dekker, Inc., New York, 1978.

Therapeutic compositions can be administered with medical devices known in the art. For example, in one embodiment, a therapeutic composition of the invention can be administered with a needleless hypodermic injection device, such as the devices shown in U.S. Pat. Nos. 5,399,163; 5,383,851; 5,312,335; 5,064,413; 4,941,880; 4,790,824 or 4,596,556. Examples of well known implants and modules useful in the present invention include: U.S. Pat. No. 4,487,603, which shows an implantable micro-infusion pump for dispensing medication at a controlled rate; U.S. Pat. No. 4,486,194, which shows a therapeutic device for administering medicants through the skin; U.S. Pat. No. 4,447,233, which shows a medication infusion pump for delivering medication at a precise infusion rate; U.S. Pat. No. 4,447,224, which shows a variable flow implantable infusion apparatus for continuous drug delivery; U.S. Pat. No. 4,439,196, which shows an osmotic drug delivery system having multi-chamber compartments; and U.S. Pat. No. 4,475,196, which shows an osmotic drug delivery system. These patents are incorporated herein by reference. Many other such implants, delivery systems, and modules are known to those skilled in the art.

In certain embodiments, the LRP6 binding molecules of the invention can be formulated to ensure proper distribution in vivo. For example, the blood-brain barrier (BBB) excludes many highly hydrophilic compounds. To ensure that the therapeutic compounds of the invention cross the BBB (if desired), they can be formulated, for example, in liposomes. For methods of manufacturing liposomes, see, e.g., U.S. Pat. Nos. 4,522,811; 5,374,548; and 5,399,331. The liposomes may comprise one or more moieties which are selectively transported into specific cells or organs, thus enhance targeted drug delivery (see, e.g., V.V. Ranade, 1989 J. Cline Pharmacol. 29:685). Exemplary targeting moieties include folate or biotin (see, e.g., U.S. Pat. 5,416,016 to Low et al.); mannosides (Umezawa et al., 1988 Biochem. Biophys. Res. Commun. 153:1038); antibodies (P.G. Bloeman et al., 1995 FEBS Lett. 357:140; M. Owais et al., 1995 Antimicrob. Agents Chernother. 39:180); surfactant protein A receptor (Briscoe et al., 1995 Am. J. Physiol.1233:134); p120 (Schreier et al., 1994 J. Biol. Chem. 269:9090); see also K. Keinanen; M.L. Laukkanen, 1994 FEBSLett. 346:123; J.J. Killion; I.J. Fidler, 1994 Immunomethods 4:273.

### Uses and methods of the invention

The LRP6 binding molecules described herein have in vitro and in vivo diagnostic and therapeutic utilities. For example, these molecules can be administered to cells in culture, e.g. in vitro or in vivo, or in a subject, e.g., in vivo, to treat, prevent or diagnose a variety of disorders. LRP6 binding molecules are particularly suitable for treating human patients suffering from "Wnt signaling-related disorders," meaning those diseases and conditions associated with aberrant Wnt signaling. Aberrant upregulation of Wnt signaling is associated with cancer, osteoarthritis, and polycystic kidney disease, which conditions would be particularly amendable to treatment by the administration of antagonizing LRP binding molecules. Conversely, aberrant downregulation of Wnt signaling has been linked to osteoporosis, obesity, diabetes, and neuronal degenerative diseases, which conditions would be particularly amendable to treatment by the administration of agonizing LRP binding molecules.

In one embodiment, LRP6 antagonizing binding molecules can be used to diagnose, ameliorate the symptoms of, protect against, and treat Wnt signaling disorders associated with aberrantly high levels of Wnt pathway by binding in a Wnt1 specific fashion, to the first propeller of LRP6. In another embodiment, LRP6 antagonizing binding molecules can be used to diagnose, ameliorate the symptoms of, protect against, and treat Wnt signaling disorders associated with aberrantly high levels of Wnt pathway by binding in a Wnt3a specific fashion, to the third propeller of LRP6. In one embodiment, LRP6 agonizing binding molecules can be used to diagnose, ameliorate the symptoms of, protect against, and treat Wnt signaling disorders associated with aberrantly low levels of Wnt pathway by binding to the third propeller of LRP6.

"Wnt signaling -related cancers" which are amenable to treatment by the administration of the LRP6 binding molecules of the invention include but are not limited to colon cancers (e.g., colorectal carcinomas (CRCs)), melanoma, breast cancer, liver cancer, lung cancer, gastric cancer, malignant medulloblastoma and other primary CNS malignant neuroectodermal tumors, rhabdomyosarcoma, gut-derived tumors (including but not limited to cancer of the esophagus, stomach, pancreas, and biliary duct system), and prostate and bladder cancers.

In a related fashion, the LRP6 antagonizing binding molecules of the invention are capable of inhibiting the growth of a tumor cell, or of inducing apoptosis in, or inhibiting angiogenesis of, a tumor cell. By way of example, a tumor cell can be contacted with an antagonizing LRP6 binding molecule (e.g., an LRP6 binding molecule including an antigen binding portion of an antibody that specifically binds to an LRP6), thereby preventing Wnt pathway signal transduction within the tumor cell via stabilizing the β-catenin destruction complex and engendering β-catenin phosphorylation and degradation.

The LRP6 binding molecules described herein are particularly suitable for treating human patients suffering from bone-related disorders, such as in the case of fractures, disorders in which bone mineral density (BMD) is abnormally and/or pathologically high relative to healthy subjects, and disorders in which bone mineral density (BMD) is abnormally and/or pathologically low relative to healthy subjects.

Disorders characterized by high BMD, which would be amenable to treatment via the administration of anti-LRP6 antagonizing binding molecules, include but are not limited to sclerosteosis, Van Buchem disease, bone overgrowth disorders, and Simpson-Golabi-Behmel syndrome (SGBS). By way of example, said antagonizing LRP6 binding molecules (e.g., an LRP6 binding molecule including an antigen binding portion of an antibody that specifically binds to an LRP6) would be administered to a subject in an amount effective to lower bone mineral density

Disorders characterized by low BMD and/or bone fragility, which would be amenable to treatment via the administration of anti-LRP6 agonizing binding molecules, include but are not limited to primary and secondary osteoporosis, osteopenia, osteomalacia, osteroporosis-pseudoglioma syndrome (OPPG), osteogenesis imperfecta (OI), avascular necrosis (osteonecrosis), fractures and implant healing (dental implants and hip implants), and bone loss due to other disorders (e.g., associated with HIV infection, cancers, or arthritis). Said agonizing LRP6 antibodies are capable of activating, enhancing, or perpetuating the Wnt signaling pathway, or sensitizing cells to Wnt signaling. Said agonizing LRP6 antibodies can bind to LRP6 (e.g., to the 3rd propeller of LRP6) and engender the dissolution of the β-catenin destruction complex, thereby permitting β-catenin stabilization, translocation to the nucleus, and engagement of transcription factors, and thereby raising bone mineral density in the process.

In some embodiments, said agonizing LRP6 binding molecules are capable of activating, enhancing, or perpetuating the Wnt signaling pathway, or sensitizing cells to Wnt signaling, by oligomerizing LRP6. It is known that the requirement for Wnt ligands can be abrogated by agents which allow formation of oligomerization of LRP6 (e.g., Fzd-LRP6 hetrero-oligomers), including the LRP6 binding molecules of the invention. (Cong et al. (2004) Development 131(20): 5103). In some embodiments, said LRP6 binding molecules are divalent IgG antibodies, as described herein.

Other "bone-related disorders" amenable to treatment via the administration of LRP6 binding molecules include but are not limited to rheumatoid arthritis, osteoarthritis, arthritis, and the formation and/or presence of osteolytic lesions.

In one embodiment, LRP6 antagonizing binding molecules can be used to diagnose, ameliorate the symptoms of, protect against, and treat bone-related disorders characterized by aberrantly high BMD by binding in a Wnt1 specific fashion, to the first propeller of LRP6. In another embodiment, LRP6 antagonizing binding molecules can be used to diagnose, ameliorate the symptoms of, protect against, and treat bone-related disorders characterized by aberrantly high BMD by binding in a Wnt3a specific fashion, to the third propeller of LRP6. In one embodiment, LRP6 agonizing binding molecules can be used to diagnose, ameliorate the symptoms of, protect against, and treat bone-related disorders characterized by aberrantly low BMD by binding to the third propeller of LRP6.

The LRP6 binding molecules described herein are particularly suitable for treating human patients suffering from diabetes, obesity, or other related metabolic disorders, e.g., those characterized by aberrantly and/or pathologically low rates of Wnt signaling relative to healthy subjects. Said disorders are amenable to treatment via the administration of anti-LRP6 agonizing binding molecules, which are capable, for example, of enhancing insulin secretion upon binding their targets. (Fujino et al. (2003) PNAS 100:229). By way of example, said agonizing LRP6 binding molecules (e.g., an LRP6 binding molecule including an antigen binding portion of an antibody that specifically binds to an LRP6) would be administered to a subject in an amount effective to enhance insulin secretion, such that a diabetic condition in said subject is prevented, ameliorated, or remedied.

In one embodiment, LRP6 agonizing binding molecules can be used to diagnose, ameliorate the symptoms of, protect against, and treat diabetes, obesity, or other related metabolic disorders (e.g., those characterized by aberrantly and/or pathologically low rates of Wnt signaling relative to healthy subjects) by binding to the third propeller of LRP6.

When LRP6 binding molecules are administered together with another agent, the two can be administered sequentially in either order or simultaneously. In some embodiments, an LRP6 binding molecule is administered to a subject who is also receiving therapy with a second agent. Said second agent can be a chemotherapeutic, in the case of cancers; an insulin secretion enhancer, such as nateglinides or repaglinides, in the case of diabetes or other metabolic disorders; and agents capable of increasing bone density, such as calcium, vitamin D, or bisphosphonates, in the case of bone-related disorders. A combination therapy regimen may be additive, or it may produce synergistic results (e.g., increases in bone mineral density, or in insulin secretion, or in apoptosis of cancer cells greater than expected for the combined use of the two agents).

In one embodiment, the binding molecules of the invention can be used to detect levels of LRP6. This can be achieved, for example, by contacting a sample (such as an in vitro sample) and a control sample with the LRP6 binding molecule under conditions that allow for the formation of a complex between the binding molecule and LRP6. Any complexes formed between the molecule and LRP6 are detected and compared in the sample and the control. For example, standard detection methods, well known in the art, such as ELISA and flow cytometric assays, can be performed using the compositions of the invention.

Accordingly, in one aspect, the invention further provides methods for detecting the presence of LRP6 (e.g., hLRP6) in a sample, or measuring the amount of LRP6, comprising contacting the sample, and a control sample, with an LRP6 binding molecule (e.g., an antibody) of the invention, under conditions that allow for formation of a complex between the antibody or portion thereof and LRP6. The formation of a complex is then detected, wherein a difference in complex formation between the sample compared to the control sample is indicative of the presence of LRP6in the sample.

Also within the scope of the invention are kits consisting of the compositions of the invention and instructions for use. The kit can further contain a least one additional reagent, or one or more additional antibodies of the invention (e.g., an antibody having a complementary activity which binds to an epitope on the target antigen distinct from the first antibody). Kits typically include a label indicating the intended use of the contents of the kit. The term label includes any writing, or recorded material supplied on or with the kit, or which otherwise accompanies the kit.

The invention having been fully described, it is further illustrated by the following examples and claims, which are illustrative and are not meant to be further limiting. Those skilled in the art will recognize or be able to ascertain using no more than routine experimentation, numerous equivalents to the specific procedures described herein. Such equivalents are within the scope of the present invention and claims. The contents of all references, including issued patents and published patent applications, cited throughout this application are hereby incorporated by reference.

### EXAMPLES

### Example 1: Identification of Wnt-Specific anti-LRP6 Antagonizing Antibodies

Anti-LRP6 antagonistic Fabs were found to preferentially inhibit Wnt1- or Wnt3a-induced Wnt signaling. 293T cells were transiently transfected with Wnt1 or Wnt3a expression plasmids together with SuperTopflash and SV40-Renilla reporters. The ratio of luciferase signal between cells treated with LRP6 Fabs and cells treated with anti-lyzosome control Fab (FabControl) was plotted and depicted in at least Figures 1 and 2.

Wnt signaling pathway proteins that are capable of canonical Wnt signaling can be divided into two classes vis-à-vis the LRP6 binding molecules of the invention. Wnt3 and Wnt3a were blocked by Wnt3a-specific LRP6 antagonistic Abs. Other Wnt proteins (Wnt1, 2, 6, 7A, 7B, 9, 10A, 10B) were blocked by Wnt1-specific LRP6 antagonistic Abs.

Experiments were performed by transiently transfecting 293T cells with different Wnt and Frizzled constructs together with SuperTopflash and SV40-Renilla, and treating with a panel of LRP6 antagonistic Fabs. The ratio of luciferase signal between cells treated LRP6 Fabs and cells treated with anti-lyzosome control Fab (FabControl) is shown in Table II:

**TABLE II**

| | **Wnt3** | **Wnt3A** | **Wnt1** | **Wnt2 +Fzd8** | **Wnt6 +Fzd8** | **Wnt7A +Fzd8** | **Wnt7B +Fzd8** | **Wnt9A +Fzd10** | **Wnt10A +Fzd5** | **Wnt10B +Fzd8** |
|---|---|---|---|---|---|---|---|---|---|---|
| **Fab003** | 2% | 3% | 64% | 76% | 66% | 89% | 79% | 49% | 87% | 84% |
| **Fab004** | 1% | 3% | 87% | 102% | 89% | 95% | 96% | 73% | 120% | 85% |
| **Fab023** | 9% | 23% | 62% | 79% | 76% | 96% | 75% | 81% | 130% | 72% |
| **Fab015** | 83% | 77% | 1% | 1% | 2% | 14% | 34% | 8% | 23% | 23% |
| **Fab016** | 73% | 76% | 1% | 0% | 2% | 13% | 30% | 5% | 10% | 19% |
| **Fab019** | 66% | 70% | 1% | 1% | 2% | 13% | 29% | 13% | 16% | 23% |
| **Fab020** | 68% | 69% | 1% | 1% | 2% | 13% | 29% | 17% | 19% | 18% |
| **FabControl** | 100% | 100% | 100% | 100% | 100% | 100% | 100% | 100% | 100% | 100% |

### Example 2: Identification of Wnt-Specific anti-LRP6 Antibodies

Anti-LRP6 agonistic Fabs were identified. 293T-STF cells were treated with indicated antibodies (1ug/ml), and 0% or 5% Wnt3a-conditioned medium overnight, and luciferase activity was measured. The ratio of luciferase signal between cells treated LRP6 Fabs and cells treated with anti-lyzosome control Fab (Fab control) at the indicated Wnt3a concentration was plotted, as seen at least in Figure 3.

### Example 3: Domain Mapping of LRP6 and Epitopic Determination

As seen in at least Figure 4, domain mapping of LRP6 deletion mutants using FACS assay reveals that Wnt1-specific LRP6 antagonistic antibodies bind to propeller 1, and Wn3a-specific LRP6 antagonistic antibodies and agonistic LRP6 antibodies bind to propeller3. 293T cells were transiently transfected with N-terminal Flag-tagged wildtype or mutant LRP6 expression constructs together with MESD. Cells were stained with anti-Flag antibody (Figure 4B) and indicated Fabs (Figure 4C) and analyzed by FACS. Red curves represent two agonistic LRP6 antibodies (Fab025 and Fab026). Purple curves represent two Wnt1 specific LRP6 antagonistic antibodies (Fab010 and Fab021). Dark blue curves represent two Wnt3a specific LRP6 antagonistic antibodies (Fab002 and Fab004). Light blue curve represents a LRP6 antagonistic antibody that inhibits both Wnt3a and Wnt1 induced signaling (Fab005). Table III shows relative binding ability between the anti-LRP6 Fabs and LRP6 truncation mutants (this tabular data corresponds to the FACS analyses figures shown in Figures 4A-4C). "--" indicates no activity, and "+" indicates activity (more symbols for more activity).

**TABLE III**

| | **Agonist** | **Wnt1 Antagonist** | **Wnt3A Antagonist** | **Wnt3A, Partial Wnt1 Antagonist** |
|---|---|---|---|---|
| **LRP6 full length** | +++ | +++ | +++ | +++ |
| **LRP6 del I** | +++ | -- | +++ | +++ |
| **LRP6 del II** | +++ | +++ | +++ | +++ |
| **LRP6 del III** | -- | +++ | -- | ++ |
| **LRP6 del I-II** | +++ | -- | +++ | +++ |
| **LRP6 del I-III** | -- | -- | -- | -- |

As described throughout the application and depicted in at least Figures 4A-4C, those Fabs capable of binding to propeller 1 of LRP6 function with Wnt1 specificity (among them, Wnt1-specific Fabs Fab010 and Fab021), whereas those Fabs binding to propeller 3 of LRP6 function with Wnt3A specificity (among them, Wnt3A-specific Fabs Fab002 and Fab004). As demonstrated herein, Wnt3- and Wnt3a-specific signaling activity can be most effectively inhibited by a Wnt3a specific LRP6 antagonizing binding molecule; and Wnt1-, Wnt2-, Wnt6-, Wnt7a-, Wnt7b-, and Wnt10-specific signaling activity can be most effectively inhibited by a Wnt1 specific LRP6 antagonizing binding molecule. Agonizing LRP6 binding molecules (e.g., Fab025 and Fab026) are not thought to be Wnt specific, and bind the LRP6 3rd propeller (i.e., they lose LRP6 binding capacity only when propeller 3 is deleted (represented in LRP6 del III)).

### Example 4: Oligomerization of LRP6 enhances Wnt signaling

As described in Figure 5, LRP6 antagonistic antibodies show agonistic activity when converted to IgG.. 293T cells were transfected with Wnt1 or Wnt3a together with SuperTopflash reporter, and treated with LRP6 antibodies. Luciferase activity was normalized against anti-lysozyme control (Fab control). Once converted to IgG, Wnt1-specific antagonistic antibodies show agonistic activity in Wnt3a assay, while Wnt3a-specific antagonistic antibodies show agonistic activity in Wnt1 assay. These observations are consistent with the observation that Wnt1 and Wnt3a bind to different regions of LRP6 for signaling, and that oligomerization of LRP6 enhances Wnt signaling. For example, Wnt1 specific antagonistic IgGs inhibit Wnt1 signaling by blocking the physical interaction between Wnt1 and LRP6. Wnt1 specific antibodies, however, do not block the Wnt3a-LRP6 interaction, and instead induce oligomerization of LRP6. This leads to enhanced response to Wnt3a ligand. These data suggest that oligomerization of endogenous LRP6 increases Wnt signaling.

### Example 5: Inhibition of LRP6 phosphorylation and free β-Catenin Accumulation

PA-1 cells are ovarian teratocarcinoma cells. These were treated with six hours' worth of stimulation with a combination of Fab004, Fab004, and Fab012. The phospho-LRP6 (pLRP6) and LRP6 samples were prepared using a Triton-X buffer whole cell lysis. The β-Catenin IB samples were prepared by cell fractionation by multiple freeze thaws in hypotonic solution.

NCI-H929 cells are multiple myeloma cells. 4 × 10⁶ cells were used. These were preincubated with protein for 30 minutes, followed by four hours' worth of Wnt3a stimulation (50% conditioned media). Cell fractionation was performed by multiple freeze thaw in hypotonic solution, and the membrane fraction re-suspended in Triton-X buffer.

## Claims

1. A bispecific molecule comprising a first antibody, or antigen-binding fragment thereof, with binding specificity for an epitope on human low density lipoprotein-related protein 6 (LRP6) and a second antibody, or antigen-binding fragment thereof, with binding specificity for a second epitope on human LRP6, wherein the bispecific molecule binds to both propeller 1 and propeller 3 of human LRP6 and antagonizes Wnt1-specific and Wnt3/Wnt3a-specific Wnt signalling.

2. The bispecific molecule of claim 1, wherein said first antigen-binding fragment and/or said second antigen-binding fragment is/are selected from a monovalent fragment consisting of the V_{L}, V_{H}, C_{L} and CH1 domains (Fab fragment); a bivalent fragment comprising two Fab fragments linked by a disulfide bridge at the hinge region (F(ab)2 fragment); an Fd fragment consisting of the V_{H} and CH1 domains; an Fv fragment consisting of the V_{L} and V_{H} domains of a single arm of an antibody; a single domain antibody (dAb) fragment which consists of a V_{H} domain; and an isolated complementarity determining region (CDR).

3. The bispecific molecule of claim 1 or 2, wherein said first antibody or antigen-binding fragment thereof and/or said second antibody or antigen binding fragment thereof is/are a VHH region of a camelid antibody.

4. The bispecific molecule of any one of claims 1-3, wherein said first antibody or fragment and/or said second antibody or fragment is/are conjugated or coupled with a human serum albumin (HSA) binding protein.

5. The bispecific molecule of any one of claims 1-4, wherein the antigen binding portion of (i) said first antibody or fragment or (ii) said second antibody or fragment binds to an epitope of human LRP6 within amino acids 20-326 of SEQ ID NO:1.

6. The bispecific molecule of any one of claims 1-4, wherein the antigen binding portion of (i) said first antibody or fragment or (ii) said second antibody or fragment binds to an epitope of human LRP6 within amino acids 631-932 of SEQ ID NO:1.

7. The bispecific molecule of any one of claims 1-6, wherein the antigen binding portion of said first antibody or fragment binds to an epitope of human LRP6 within amino acids 20-326 of SEQ ID NO:1 and the antigen binding portion of said second antibody or fragment binds to an epitope of human LRP6 within amino acids 631-932 of SEQ ID NO:1 or wherein the antigen binding portion of said first antibody or fragment binds to an epitope of human LRP6 within amino acids 631-932 of SEQ ID NO:1 and the antigen binding portion of said second antibody or fragment binds to an epitope of human LRP6 within amino acids 20-326 of SEQ ID NO:1.

8. The bispecific molecule of any one of claims 1-7, wherein said bispecific molecule is a single chain molecule.

9. An isolated nucleic acid encoding the bispecific molecule of claim 8.

10. A vector comprising the isolated nucleic acid of claim 9.

11. A host cell comprising the vector of claim 10.

12. The bispecific molecule of any one of claims 1-8, formulated together with a pharmaceutically acceptable carrier.

13. The bispecific molecule of any one of claims 1-8 for use in the treatment of cancer, wherein said cancer is associated with upregulation of Wnt signalling.

14. The bispecific molecule for used according to claim 13, wherein the cancer is selected from the group consisting of colorectal carcinoma (CRCs), melanoma, breast cancer, liver cancer, lung cancer, gastric cancer, malignant medulloblastoma or other primary CNS malignant neuroectodermal tumor, rhabdomyosarcoma, a gut-derived tumor (e.g. cancer of the esophagus, stomach cancer, pancreatic cancer, and biliary duct system cancer), prostate cancer, bladder cancer, colon cancer, leukemia (e.g. Chronic Lymphocytic Leukemia (CLL), Acute Myelogenous Leukemia (AML), Acute Lymphocytic Leukemia (ALL), Chronic Myelogenous Leukemia (CML), and Myelodysplastic Syndromes (MOS)), and lymphoma (e.g., Hodgkin lymphoma and non-Hodgkin lymphoma).

15. The bispecific molecule for use according to claims 13 or 14, wherein the bispecific molecule is administered intravenously.

16. The bispecific molecule for use according to any one of claims 13-15, wherein the bispecific molecule is administered with another therapeutic agent.
